# EUROPEAN PATENT APPLICATION

(11) **EP 3 978 004 A1**
(43) Date of publication of application: **06.04.2022**
(21) Application number: 20199715.2
(22) Date of filing: 01.10.2020
(51) Int. Cl.: A61K 35/64, A61K 38/06, A61K 38/17, A61P 31/02, A61P 31/04, A61P 31/10, A01N 63/14

(54) **A COMPOSITION FOR USE AS A BACTERICIDE**

(71) Applicant: Toggam Enterprises Limited, County Meath (IE)
(72) Inventor: Alvan, HUNT, Co. Meath (IE); John, LYNAM, Co. Meath (IE); Kevin, KAVANAGH, Co. Meath (IE); Anatte, MARGALIT, Co. Meath (IE)
(74) Representative: FRKelly

(57) **Abstract**

The present invention relates to a composition for use as a bactericide. The composition finds utility as a disinfectant, antiseptic, or antibiotic. The composition comprises Hermetia Illucens or derivative compounds therefrom.

## Description

### Field of the Invention

The present invention relates to a composition for use as a bactericide. The composition finds utility as a disinfectant, antiseptic, or antibiotic. The composition comprises Hermetia Illucens or derivative compounds therefrom.

### Background to the Invention

Bacterial infections contribute up to 54% of global infectious diseases, which is why antibiotics are so commonly used.

Escherichia coli (E coli) is a gram negative, rod-shaped facultative anaerobic bacterium with a rapid reproductive capacity. E coli are commonly found in the human gastrointestinal tract and can cause a variety of health issues in the event of overpopulation. E coli is most commonly responsible for urinary tract infections and is the bacterium commonly responsible for diarrhoea in people travelling long distances. E coli is a common pathogen in foodborne infections, which can have severe health consequences particularly on young children.

Pseudomonas aeruginosa (P. aeruginosa) is an environmental gram negative bacterium with a high affinity for the infection of the lungs of patients with cystic fibrosis. Chronic infection occurs in over 60% of adults with cystic fibrosis. Chronic infection is associated with accelerated disease progression and increased mortality. The P. aeruginosa capsule, composed of polysaccharide alginate, is an important virulence factor encountered primarily in cystic fibrosis. The regulatory algR gene positively controls transcription of a key alginate biosynthetic gene, algD.

Staphylococcus aureus (Staph aureus) is a gram positive bacterium that can be found in 25% of humans and it can commonly cause illness through direct infection or through toxins produced by the bacterial cells. Staph aureus can cause a variety of community and hospital-associated pathologies, such as bacteraemia, sepsis, endocarditis, pneumonia, osteomyelitis, arthritis and skin diseases. S. *aureus* has also become a much more dangerous threat to human health as the antibiotic resistant strain known as MRSA (methicillin resistant Staphylococcus aureus) is much more difficult to treat. MRSA can produce high rates of morbidity and mortality and can cause metastatic or complicated infections such as infective endocarditis or sepsis. MRSA is responsible for most global S. *aureus* bacteraemia cases, and compared with methicillin-sensitive S. *aureus,* MRSA infection is associated with poorer clinical outcomes. S. *aureus* virulence is affected by the unique combination of toxin and immune-modulatory gene products.

Multiple drug resistance has been identified as one of the biggest problems facing medical research today. This is due to the rapid rate at which microorganisms are evolving in order to survive the onslaught of antibiotics since the discovery of penicillin in 1928. It has been estimated that every year in Europe over 25,000 people die due to a multidrug resistant microorganism infection and the European Union spends over 1.5 billion euro annually to combat this issue which is progressively increasing.

It has been said that we are approaching the saturation point of reliable antibiotic use and that we may be entering a period with a lack of infection control similar to that of the time before the discovery of antibiotic three quarters of a century ago. Alternatives are currently under investigation.

There is a need to provide a novel composition for use as a disinfectant, antiseptic, or antibiotic.

### Summary of the Invention

According to a first aspect of the present invention, there is provided a composition comprising Hermetia Illucens or derivative compounds therefrom for use as a bactericide.

According to a second aspect of the present invention, there is provided use of a composition comprising Hermetia Illucens or derivative compounds therefrom as a bactericide.

According to a third aspect of the present invention, there is provided a composition comprising Hermetia Illucens or derivative compounds therefrom for use as a disinfectant, antiseptic, or antibiotic.

According to a fourth aspect of the present invention, there is provided use of a composition comprising Hermetia Illucens or derivative compounds therefrom as a disinfectant, antiseptic, or antibiotic.

According to a fifth aspect of the present invention, there is provided a composition comprising Hermetia Illucens or derivative compounds therefrom for use in the treatment of a bacterial infection.

Optionally, the use comprises administering the composition comprising Hermetia Illucens or derivative compounds therefrom to the bacterial infection. Further optionally, the use comprises topically administering the composition comprising Hermetia Illucens or derivative compounds therefrom to the bacterial infection.

According to a sixth aspect of the present invention, there is provided use of Hermetia Illucens or derivative compounds therefrom in the manufacture of a medicament for treatment of a bacterial infection.

According to a seventh aspect of the present invention, there is provided a method for treatment of a bacterial infection, the method comprising the step of administering a composition comprising Hermetia Illucens or derivative compounds therefrom to the bacterial infection.

Optionally, the method comprises the step of topically administering a composition comprising Hermetia Illucens or derivative compounds therefrom to the bacterial infection.

According to an eighth aspect of the present invention, there is provided a disinfectant, antiseptic, or antibiotic composition comprising Hermetia Illucens or derivative compounds therefrom.

According to a ninth aspect of the present invention, there is provided a method of preparing a disinfectant, antiseptic, or antibiotic composition comprising Hermetia Illucens or derivative compounds therefrom.

According to a tenth aspect of the present invention, there is provided a disinfectant, antiseptic, or antibiotic composition obtained by the method of preparing the antibiotic composition according to a ninth aspect of the present invention.

Optionally, the bacterium is a gram-negative bacterium. Alternatively, the bacterium is a gram-positive bacterium.

Optionally, the bacterium is selected from the genus Staphylococcus, Pseudomonas, Salmonella, Bacillus, Escherichia, and/or Klebsiella. Further optionally, the bacterium is selected from Staphylococcus aureus, methicillin resistant Staphylococcus aureus (MRSA), Staphylococcus Epidermidis, Pseudomonas aeruginosa, Salmonella spp., Salmonella typhimurium, Salmonella maltophilia, Bacillus subtilis, Escherichia coli, Escherichia meningoseptica, and Klebsiella pneumonia.

Optionally, there is provided a composition comprising Hermetia Illucens or derivative compounds therefrom for use as a fungicide.

Optionally, there is provided use of a composition comprising Hermetia Illucens or derivative compounds therefrom as a fungicide.

Optionally, there is provided a composition comprising Hermetia Illucens or derivative compounds therefrom for use in the treatment of a fungal infection.

Optionally, there is provided use of Hermetia Illucens or derivative compounds therefrom in the manufacture of a medicament for treatment of a fungal infection.

Optionally, there is provided a method for treatment of a fungal infection, the method comprising the step of administering a composition comprising Hermetia Illucens or derivative compounds therefrom.

Optionally, the fungus is selected from the genus Candida, Aspergillus, and/or Penicillium. Further optionally, the fungus is selected from Candida albicans, Candida glabrata, Candida krusei, Aspergillus fumigatus, and Penicillium spp.

Optionally, the composition comprises Hermetia Illucens. Further optionally, the composition comprises mature Hermetia Illucens. Optionally or additionally, the composition comprises Hermetia Illucens larvae. Optionally or additionally, the composition comprises Hermetia Illucens larval cuticle.

Preferably, the composition comprises Hermetia Illucens larval cuticle.

Optionally, the composition comprises dehydrated Hermetia Illucens. Further optionally, the composition comprises dehydrated mature Hermetia Illucens. Optionally or additionally, the composition comprises dehydrated Hermetia Illucens larvae. Optionally or additionally, the composition comprises dehydrated Hermetia Illucens larval cuticle.

Preferably, the composition comprises dehydrated Hermetia Illucens larval cuticle.

Optionally, the composition comprises processed dehydrated Hermetia Illucens. Further optionally, the composition comprises processed dehydrated mature Hermetia Illucens. Optionally or additionally, the composition comprises processed dehydrated Hermetia Illucens larvae. Optionally or additionally, the composition comprises processed dehydrated Hermetia Illucens larval cuticle.

Preferably, the composition comprises processed dehydrated Hermetia Illucens larval cuticle.

Optionally, the composition comprises ground dehydrated Hermetia Illucens. Further optionally, the composition comprises ground dehydrated mature Hermetia Illucens.

Optionally or additionally, the composition comprises ground dehydrated Hermetia Illucens larvae. Optionally or additionally, the composition comprises ground dehydrated Hermetia Illucens larval cuticle.

Optionally or additionally, the composition comprises Hermetia Illucens haemolymph.

Optionally or additionally, the composition comprises dehydrated Hermetia Illucens haemolymph.

Optionally or additionally, the composition comprises ground dehydrated Hermetia Illucens haemolymph.

Optionally or additionally, the composition comprises Hermetia Illucens frass.

Optionally or additionally, the composition comprises dehydrated Hermetia Illucens frass.

Optionally or additionally, the composition comprises ground dehydrated Hermetia Illucens frass.

Optionally, the composition comprises at least one peptide. Further optionally, the composition comprises at least one Hermetia Illucens peptide. Still further optionally, the composition comprises at least one peptide derived from Hermetia Illucens.

Preferably, the composition comprises at least one peptide derived from Hermetia Illucens.

Optionally, the at least one peptide is less than 200 amino acids in length. Further optionally, the at least one peptide is less than 190, optionally less than 180, optionally less than 170, optionally less than 160, optionally less than 150, optionally less than 140, optionally less than 130, optionally less than 120, optionally less than 110, optionally less than 100, optionally less than 90, optionally less than 80, optionally less than 700, optionally less than 60, optionally less than 50 amino acids in length.

Preferably, the at least one peptide is less than 100 amino acids in length.

Optionally, the peptide is an anionic peptide. Alternatively, the peptide is a cationic peptide.

Preferably, the peptide is a cationic peptide.

Optionally, the composition comprises a protein selected from phenoloxidase 2; chitinase-like protein Idgfl; angiotensin-converting enzyme; ferritin; transferrin; RE42475p; cystathionine beta-synthase; heat shock protein 83; tubulin beta-1 chain; ejaculatory bulb-specific protein 3; and tubulin alpha-1 chain.

Optionally or additionally, the composition comprises a protein selected from actin-5C; phenoloxidase 3; thioester-containing protein 2, isoform G; fumarase 1, isoform B; CathD, isoform A; FI17510pl; fructe-bisphphate aldolase; nucleome remodeling factor- 38kD, isoform C; myosin heavy chain, isoform S; and aldehyde dehydrogenase.

Optionally or additionally, the composition comprises a protein selected from actin-42A; thioester-containing protein 2, isoform F; myosin heavy chain, isoform V; heat shock 70 kDa protein cognate 4; glyceraldehyde-3-phosphate dehydrogenase 1; elongation factor 1 -alpha 1; major heat shock 70 kDa protein Ba; FI05334p; enolase; and chromatin-remodelling A TPase IN080.

Optionally, the composition comprises at least one peptide.

Optionally, the peptide comprises at least one amino acid. Further optionally, the peptide comprises at least two, optionally at least three, optionally at least four, optionally at least five, optionally at least six amino acids.

Preferably, the peptide comprises at least three amino acids.

Optionally or additionally, the peptide comprises at least one carbohydrate. Further optionally or additionally, the peptide comprises at least one saccharide molecule. Still further optionally or additionally, the peptide comprises at least one monosaccharide molecule.

Preferably, the peptide comprises at least one monosaccharide molecule.

Preferably, the peptide comprises at least three amino acids and at least one monosaccharide molecule.

Optionally, the at least one amino acid is a non-polar amino acid. Further optionally, the at least one amino acid is a non-polar amino acid selected from glycine, alanine, valine, leucine, isoleucine, proline, phenylalanine, methionine, and tryptophan.

Preferably, the at least one amino acid is a non-polar amino acid selected from alanine.

Optionally, the at least one amino acid is a polar amino acid. Further optionally, the at least one amino acid is a polar amino acid selected from serine, cysteine, threonine, tyrosine, asparagine, and glutamine. Still further optionally, the at least one amino acid is a polar amino acid selected from serine and threonine.

Preferably, the at least one amino acid is a polar amino acid selected from serine.

Optionally, the peptide comprises at least one alanine residue and at least one serine residue. Further optionally, the peptide comprises at least two alanine residues and at least one serine residue. Alternatively, the peptide comprises at least one alanine residue and at least two serine residues.

Preferably, the peptide comprises two alanine residues and one serine residue. Further preferably, the peptide comprises two alanine residues and one serine residue and at least one monosaccharide molecule. Still further preferably, the peptide comprises two alanine residues and one serine residue and at least one monosaccharide molecule, wherein the at least one monosaccharide molecule is attached to the serine residue. Still further preferably, the peptide comprises two alanine residues and one serine residue and at least one monosaccharide molecule, wherein the at least one monosaccharide molecule is covalently attached to the serine residue. Still further preferably, the peptide comprises two alanine residues and one serine residue and at least one monosaccharide molecule, wherein each alanine residue is attached to the one serine residue, and wherein the at least one monosaccharide molecule is attached to the serine residue. Still further preferably, the peptide comprises two alanine residues and one serine residue and at least one monosaccharide molecule, wherein each alanine residue is covalently attached to the one serine residue, and wherein the at least one monosaccharide molecule is attached to the serine residue.

Preferably, the peptide comprises alanine-serine-alanine and at least one monosaccharide molecule, wherein the at least one monosaccharide molecule is attached to the serine residue.

Optionally, the monosaccharide molecule is a sugar molecule.

Optionally, the monosaccharide molecule is a hexose molecule. Further optionally, the monosaccharide molecule is a hexose molecule selected from allose, altrose, glucose, mannose, gulose, idose, galactose, and talose. Still further optionally, the monosaccharide molecule is a d-hexose molecule selected from d-allose, d-altrose, d-glucose, d-mannose, d-gulose, d-idose, d-galactose, and d-talose.

Preferably, the monosaccharide molecule is mannose. Further preferably, the monosaccharide molecule is d-mannose.

Preferably, the peptide comprises alanine-serine-alanine and at least one mannose molecule, wherein the at least one mannose molecule is attached to the serine residue.

Optionally, the monosaccharide molecule is an amino sugar molecule. Still further optionally, the monosaccharide molecule is a 2-amino-2-deoxysugar molecule. Still further optionally, the monosaccharide molecule is mannosamine (2-amino-2-deoxymannose).

Preferably, the monosaccharide molecule is mannosamine.

Preferably, the peptide comprises alanine-serine-alanine and at least one mannosamine molecule, wherein the at least one mannosamine molecule is attached to the serine residue.

Alternatively, the monosaccharide molecule is glucose. Further preferably, the monosaccharide molecule is d-glucose.

Preferably, the peptide comprises alanine-serine-alanine and at least one glucose molecule, wherein the at least one glucose molecule is attached to the serine residue.

Optionally, the monosaccharide molecule is an amino sugar molecule. Still further optionally, the monosaccharide molecule is a 2-amino-2-deoxysugar molecule. Still further optionally, the monosaccharide molecule is glucosamine (2-Amino-2-deoxy-glucose).

Preferably, the monosaccharide molecule is glucosamine.

Preferably, the peptide comprises alanine-serine-alanine and at least one glucosamine molecule, wherein the at least one glucosamine molecule is attached to the serine residue.

Optionally, the method of preparing a disinfectant, antiseptic, or antibiotic composition comprises the steps of:
(a) providing Hermetia Illucens;
(b) dehydrating the Hermetia Illucens; and
(c) processing the dehydrated Hermetia Illucens.

Optionally, the method comprises providing mature Hermetia Illucens. Optionally or additionally, the method comprises providing Hermetia Illucens larvae. Optionally or additionally, the method comprises providing Hermetia Illucens larval cuticle.

Preferably, the method comprises providing Hermetia Illucens larval cuticle.

Optionally, the method comprises dehydrating Hermetia Illucens. Further optionally, the method comprises dehydrating mature Hermetia Illucens. Optionally or additionally, the method comprises dehydrating Hermetia Illucens larvae. Optionally or additionally, the method comprises dehydrating Hermetia Illucens larval cuticle.

Preferably, the method comprises dehydrating Hermetia Illucens larval cuticle.

Optionally, the method comprises processing the dehydrated Hermetia Illucens. Further optionally, the method comprises processing the dehydrated mature Hermetia Illucens. Optionally or additionally, the method comprises processing the dehydrated Hermetia Illucens larvae. Optionally or additionally, the method comprises processing the dehydrated Hermetia Illucens larval cuticle.

Preferably, the method comprises processing the dehydrated Hermetia Illucens larval cuticle.

Optionally, the method comprises grinding the dehydrated Hermetia Illucens. Further optionally, the method comprises grinding the dehydrated mature Hermetia Illucens.

Optionally or additionally, the method comprises grinding the dehydrated Hermetia Illucens larvae. Optionally or additionally, the method comprises grinding the dehydrated Hermetia Illucens larval cuticle.

Optionally or additionally, the method comprises providing Hermetia Illucens haemolymph.

Optionally or additionally, the method comprises dehydrating Hermetia Illucens haemolymph.

Optionally or additionally, the method comprises processing dehydrated Hermetia Illucens haemolymph.

Optionally or additionally, the method comprises grinding dehydrated Hermetia Illucens haemolymph.

Optionally or additionally, the method comprises providing Hermetia Illucens frass.

Optionally or additionally, the method comprises dehydrating Hermetia Illucens frass.

Optionally or additionally, the composition comprises processing dehydrated Hermetia Illucens frass.

Optionally or additionally, the composition comprises grinding dehydrated Hermetia Illucens frass.

Optionally, the method of preparing a disinfectant, antiseptic, or antibiotic composition further comprises the step of stressing the Hermetia Illucens. Further optionally, the method of preparing a disinfectant, antiseptic, or antibiotic composition further comprises the step of stressing the Hermetia Illucens after the step of providing Hermetia Illucens.

Optionally, the method of preparing a disinfectant, antiseptic, or antibiotic composition comprises the steps of:
(a) providing Hermetia Illucens;
(b) stressing the Hermetia Illucens;
(c) dehydrating the stressed Hermetia Illucens; and
(d) processing the dehydrated Hermetia Illucens.

Optionally, the step of stressing the Hermetia Illucens comprises physically stressing the Hermetia Illucens.

Optionally, the step of stressing the Hermetia Illucens comprises agitating the Hermetia Illucens. Further optionally, the step of stressing the Hermetia Illucens comprises shaking the Hermetia Illucens.

Optionally, the step of stressing the Hermetia Illucens comprises changing the temperature of the Hermetia Illucens. Further optionally, the step of stressing the Hermetia Illucens comprises changing the temperature of the Hermetia Illucens to above or below 28°C. Still further optionally, the step of stressing the Hermetia Illucens comprises changing the temperature of the Hermetia Illucens to less than 28°C, optionally less than 26°C, optionally less than 24°C, optionally less than 22°C, optionally less than 20°C, optionally less than 18°C, optionally less than 16°C, optionally less than 14°C, optionally less than 12°C, optionally less than 10°C, optionally less than 8°C, optionally less than 6°C, optionally less than 4°C, optionally less than 2°C, optionally less than 0°C.

Alternatively, the step of stressing the Hermetia Illucens comprises changing the temperature of the Hermetia Illucens to greater than 28°C, optionally greater than 30°C, optionally greater than 32°C, optionally greater than 34°C, optionally greater than 36°C, optionally greater than 38°C, optionally greater than 40°C, optionally greater than 42°C, optionally greater than 44°C, optionally greater than 46°C, optionally greater than 48°C, optionally greater than 50°C.

Optionally, the step of stressing the Hermetia Illucens is conducted for at least 10 seconds. Further optionally, the step of stressing the Hermetia Illucens is conducted for at least 20 seconds, optionally at least 30 seconds, optionally at least 40 seconds, optionally at least 50 seconds, optionally at least 60 seconds, optionally at least 70 seconds, optionally at least 80 seconds, optionally at least 90 seconds, optionally at least 100 seconds, optionally at least 110 seconds, optionally at least 120 seconds, optionally at least 130 seconds, optionally at least 140 seconds, optionally at least 150 seconds, optionally at least 160 seconds, optionally at least 170 seconds, optionally at least 180 seconds.

Preferably, the step of stressing the Hermetia Illucens is conducted for at least 120 seconds.

Optionally, the method the method of preparing a disinfectant, antiseptic, or antibiotic composition further comprises the step of fractionating the processed Hermetia Illucens

Optionally, the method of preparing a disinfectant, antiseptic, or antibiotic composition comprises the steps of:
(a) providing Hermetia Illucens;
(b) dehydrating the stressed Hermetia Illucens;
(c) processing the dehydrated Hermetia Illucens; and
(d) fractionating the processed Hermetia Illucens.

Further optionally, the method of preparing a disinfectant, antiseptic, or antibiotic composition comprises the steps of:
(a) providing Hermetia Illucens;
(b) stressing the Hermetia Illucens;
(c) dehydrating the stressed Hermetia Illucens;
(d) processing the dehydrated Hermetia Illucens; and
(e) fractionating the processed Hermetia Illucens.

Optionally, the fractionating step comprises the step of removing lipids from the Hermetia Illucens.

Optionally, the fractionating step comprises the step of contacting the Hermetia Illucens with a hydrocarbon to form a hydrocarbon suspension. Further optionally, the method comprises the step of contacting the Hermetia Illucens larva with a hydrocarbon to form a hydrocarbon suspension. Still further optionally, the method comprises the step of contacting the Hermetia Illucens larval cuticle with a hydrocarbon to form a hydrocarbon suspension.

Optionally the hydrocarbon is an acyclic hydrocarbon. Further optionally the hydrocarbon is a linear hydrocarbon. Still further optionally the hydrocarbon is a straight-chain linear hydrocarbon. Still further optionally, the hydrocarbon is an alkane. Still further optionally, the hydrocarbon is hexane.

Preferably, the hydrocarbon is hexane.

Optionally, the Hermetia Illucens is contacted with the hydrocarbon in a (w/v) ratio of at least 1:1. Further optionally, the Hermetia Illucens is contacted with the hydrocarbon in a (w/v) ratio of at least 2:1, optionally at least 3:1, optionally at least 4:1, optionally at least 5:1, optionally at least 6:1, optionally at least 7:1, optionally at least 8:1, optionally at least 9:1, optionally at least 10:1, optionally at least 11:1, optionally at least 12:1, optionally at least 13:1, optionally at least 14:1, optionally at least 15:1, optionally at least 16:1, optionally at least 17:1, optionally at least 18:1.

Preferably, the Hermetia Illucens is contacted with the hydrocarbon in a (w/v) ratio of at least 9:1.

Optionally, the fractionating step further comprises the step of adding at least one protease inhibitor to the hydrocarbon suspension.

Optionally, the contacting step is conducted for at least 1 hour. Further optionally, the contacting step is conducted for at least 2 hours, optionally at least 4 hours, optionally at least 8 hours, optionally at least 12 hours, optionally at least 18 hours, optionally at least 24 hours.

Preferably, the contacting step is conducted for at least 18 hours.

Optionally, the contacting step is conducted at a temperature of at least 1°C. Optionally, the contacting step is conducted at a temperature of at least 2°C, optionally at least 3°C, optionally at least 4°C, optionally at least 5°C, optionally at least 10°C, optionally at least 15°C, optionally at least 20°C, optionally at least 25°C, optionally at least 30°C, optionally at least 35°C, optionally at least 40°C, optionally at least 45°C, optionally at least 50°C.

Preferably, the contacting step is conducted at a temperature of at least 4°C.

Optionally, the fractionating step further comprises the step of agitating the hydrocarbon suspension. Further optionally, the fractionating step further comprises the step of homogenising the hydrocarbon suspension. Further optionally, the fractionating step further comprises the step of sonicating the hydrocarbon suspension.

Optionally, the fractionating step further comprises the step of removing the hydrocarbon from the hydrocarbon suspension.

Optionally, the fractionating step comprises the step of contacting the Hermetia Illucens with an alcohol to form an alcohol suspension. Further optionally, the fractionating step comprises the step of contacting the Hermetia Illucens larva with an alcohol to form an alcohol suspension. Still further optionally, the fractionating step comprises the step of contacting the Hermetia Illucens larval cuticle with an alcohol to form an alcohol suspension.

Optionally, the alcohol is a short-chain alcohol. Further optionally, the alcohol is a C1-C5 alcohol. Still further optionally, the alcohol is selected from methanol, ethanol, propanol, butanol, and pentanol.

Preferably, the alcohol is methanol.

Optionally, the fractionating step comprises the step of contacting the Hermetia Illucens with an alcohol solution to form an alcohol suspension. Further optionally, the fractionating step comprises the step of contacting the Hermetia Illucens larva with an alcohol solution to form an alcohol suspension. Still further optionally, the fractionating step comprises the step of contacting the Hermetia Illucens larval cuticle with an alcohol solution to form an alcohol suspension.

Preferably, the alcohol solution is an aqueous alcohol solution.

Optionally, the alcohol solution is an aqueous alcohol solution in a (v/v) ratio of at least 1:1. Further optionally, the alcohol solution is an aqueous alcohol solution in a (v/v) ratio of at least 2:1, optionally at least 3:1, optionally at least 4:1, optionally at least 5:1, optionally at least 6:1, optionally at least 7:1, optionally at least 8:1, optionally at least 9:1, optionally at least 10:1, optionally at least 11:1, optionally at least 12:1, optionally at least 13:1, optionally at least 14:1, optionally at least 15:1, optionally at least 16:1, optionally at least 17:1, optionally at least 18:1.

Preferably, the alcohol solution is an aqueous alcohol solution in a (v/v) ratio of at least 9:1.

Optionally, the fractionating step further comprises the step of adding at least one protease inhibitor to the alcohol suspension.

Optionally, the contacting step is conducted for at least 1 hour. Further optionally, the contacting step is conducted for at least 2 hours, optionally at least 4 hours, optionally at least 8 hours, optionally at least 12 hours, optionally at least 18 hours, optionally at least 24 hours.

Preferably, the contacting step is conducted for at least 18 hours.

Optionally, the contacting step is conducted at a temperature of at least 1°C. Optionally, the contacting step is conducted at a temperature of at least 2°C, optionally at least 3°C, optionally at least 4°C, optionally at least 5°C, optionally at least 10°C, optionally at least 15°C, optionally at least 20°C, optionally at least 25°C, optionally at least 30°C, optionally at least 35°C, optionally at least 40°C, optionally at least 45°C, optionally at least 50°C.

Preferably, the contacting step is conducted at a temperature of at least 4°C.

Optionally, the fractionating step further comprises the step of agitating the alcohol suspension. Further optionally, the fractionating step further comprises the step of homogenising the alcohol suspension. Further optionally, the fractionating step further comprises the step of sonicating the alcohol suspension.

Optionally, the fractionating step further comprises the step of retaining the alcohol or alcohol solution from the alcohol suspension.

Optionally, the retaining step comprises separating the alcohol or alcohol solution from the alcohol suspension. Further optionally, the retaining step comprises separating the liquid alcohol or alcohol solution phase from the solid phase of the alcohol suspension. Still further optionally, the retaining step comprises separating the liquid alcohol or alcohol solution phase from the solid phase of the alcohol suspension by centrifugation.

Optionally, the fractionating step further comprises the step of dehydrating the alcohol solution. Further optionally, the fractionating step further comprises the step of dehydrating the liquid alcohol or alcohol solution phase. Still further optionally, the fractionating step further comprises the step of dehydrating the retained liquid alcohol or alcohol solution phase.

Optionally, the dehydrating step is conducted under vacuum.

Optionally, the dehydrating step is conducted for at least 5 mins. Further optionally, the dehydrating step is conducted for at least 10 mins, optionally at least 15 mins, optionally at least 20 mins, optionally at least 25mins, optionally at least 30 mins, optionally at least 35 mins, optionally at least 40 mins, optionally at least 45 mins, optionally at least 50 mins, optionally at least 55 mins , optionally at least 60 mins.

Preferably, the dehydrating step is conducted for at least 20 mins.

Optionally, the dehydrating step is conducted at a temperature of at least 20°C. Optionally, the dehydrating step is conducted at a temperature of at least 25°C, optionally at least 30°C, optionally at least 35°C, optionally at least 40°C, optionally at least 45°C, optionally at least 50°C, optionally at least 55°C, optionally at least 60°C, optionally at least 65°C, optionally at least 70°C, optionally at least 75°C.

Preferably, the dehydrating step is conducted at a temperature of at least 55°C.

Optionally, the fractionating step further comprises the step of suspending the dehydrated alcohol solution. Further optionally, the fractionating step further comprises the step of suspending the dehydrated alcohol solution in an alcohol solution.

Preferably, the alcohol solution is an aqueous alcohol solution.

Optionally, the alcohol solution is an aqueous alcohol solution in a (v/v) ratio of at least 1:1 (water:alcohol). Further optionally, the alcohol solution is an aqueous alcohol solution in a (v/v) ratio of at least 1:2, optionally at least 1:3, optionally at least 1:4, optionally at least 1:5, optionally at least 1:6, optionally at least 1:7, optionally at least 1:8, optionally at least 1:9, optionally at least 1:10, optionally at least 1:11, optionally at least 1:12, optionally at least 1:13, optionally at least 1:14, optionally at least 1:15, optionally at least 1:16, optionally at least 1:17, optionally at least 1:18.

Preferably, the alcohol solution is an aqueous alcohol solution in a (v/v) ratio of at least 1:9.

Optionally, the alcohol is a short-chain alcohol. Further optionally, the alcohol is a C1-C5 alcohol. Still further optionally, the alcohol is selected from methanol, ethanol, propanol, butanol, and pentanol.

Preferably, the alcohol is methanol.

Optionally, the fractionating step further comprises the step of filtering the suspended alcohol solution. Further optionally, the fractionating step further comprises the step of filtering the suspended alcohol solution to a particle size of less than 1.0 micrometres. Still further optionally, the fractionating step further comprises the step of filtering the suspended alcohol solution to a particle size of less than 0.8 micrometres, optionally less than 0.6 micrometres, optionally less than 0.4 micrometres, optionally less than 0.2 micrometres.

Preferably, the fractionating step further comprises the step of filtering the suspended alcohol solution to a particle size of less than 0.2 micrometres.

Optionally, the fractionating step further comprises the step of contacting the suspended alcohol solution with an alkylsilane. Further optionally, the fractionating step further comprises the step of contacting the suspended alcohol solution with a linear alkylsilane. Still further optionally, the fractionating step further comprises the step of contacting the suspended alcohol solution with a linear C1-C18 alkylsilane. Still further optionally, the fractionating step further comprises the step of contacting the suspended alcohol solution with a linear C18 alkylsilane.

Optionally, the contacting step is conducted in the presence of a solvent. Further optionally, the contacting step is conducted in the presence of a non-polar solvent. Still further optionally, the contacting step is conducted in the presence of acetonitrile. Still further optionally, the contacting step is conducted in the presence of increasing concentrations of acetonitrile over time. Still further optionally, the contacting step is conducted in the presence of increasing concentrations of acetonitrile in water over time.

Optionally, the contacting step is conducted for 0.5 - 20.5 minutes. Further optionally, the contacting step is conducted for 1.0 - 20.0 minutes, optionally 1.5 - 19.5, optionally 2.0 - 19.0, optionally 2.5 - 18.5, optionally 3.0 - 18.0, optionally 3.5 - 17.5, optionally 4.0 - 17.0, optionally 4.5 - 16.5, optionally 5.5 - 16.0, optionally 6.0 - 15.5, optionally 6.5 - 15.0, optionally 7.0 - 14.5, optionally 7.5 - 14.0, optionally 8.0 - 13.5, optionally 8.5 - 13.0, optionally 9.0 - 12.5, optionally 9.5 - 12.0, optionally 10.0 - 11.5, optionally 10.5 - 11.0 minutes.

Optionally, the contacting step is conducted for 1.5 - 6.5 minutes. Alternatively, the contacting step is conducted for 15.0 - 16.5 minutes.

Optionally, the fractionating step further comprises the step of filtering the fractionated alcohol solution. Further optionally, the fractionating step further comprises the step of filtering the fractionated alcohol solution to a molecular weight of less than 20 kDa. Still further optionally, the method further comprises the step of filtering the fractionated alcohol solution to a particle size of less than 18 kDa, optionally less than 16 kDa, optionally less than 14 kDa, optionally less than 12 kDa, optionally less than 10 kDa, optionally less than 8 kDa, optionally less than 6 kDa, optionally less than 4kDa.

Preferably, the fractionating step further comprises the step of filtering the fractionated alcohol solution to a molecular weight of less than 10 kDa.

### Brief Description of the Drawings

Embodiments of the present invention will be described with reference to the following non-limiting examples and the accompanying drawings in which:
**Figure 1** illustrates the effect of an oil produced by pressing Hermetia illucens;
**Figure 2** illustrates the effect of an extraction of haemolymph from live Hermetia illucens;
**Figures 3** and 4 each illustrate the effect of a frass that contains Hermetia illucens;
**Figures 5-9** each illustrate the effect of a composition comprising Hermetia illucens;
**Figure 10** illustrates the effect of a composition comprising Hermetia illucens (ground, dehydrated Hermetia illucens meal) (A) and (B) and frass (F) derived from Hermetia illucens;

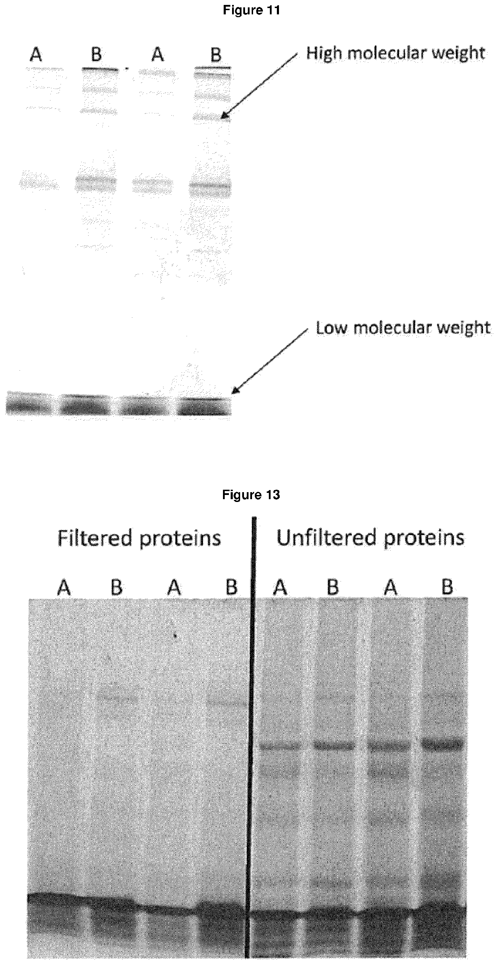

**Figure 11** illustrates high molecular weight proteins by 1 dimensional (1 D) gel electrophoresis;

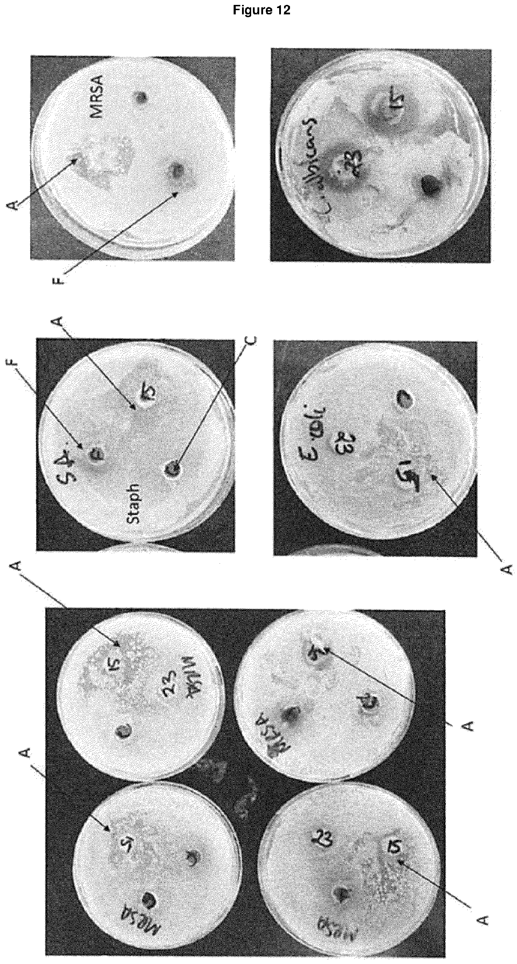

**Figure 12** illustrates the effect of proteins extracted from a composition comprising Hermetia illucens on a number of Gram-positive and Gram-negative bacteria;
**Figure 13** illustrates protein extracted from a composition comprising Hermetia illucens that were subjected to extra purification steps performed by centrifugation and filtration;

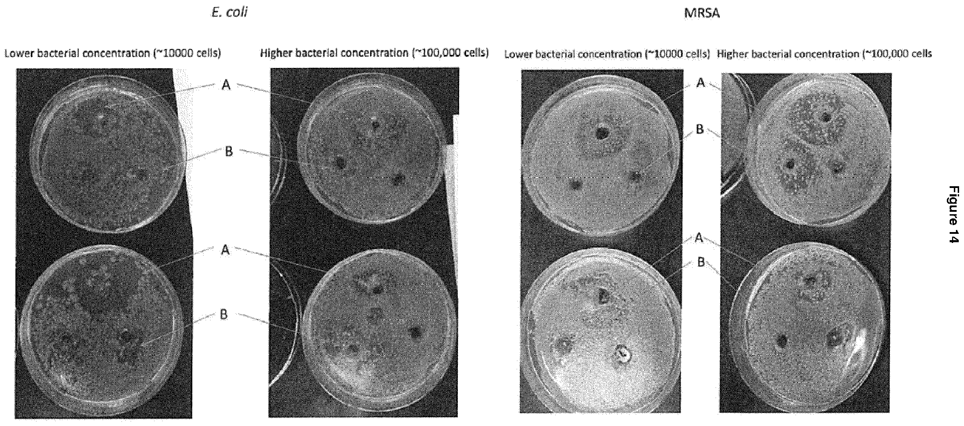

**Figure 14** illustrates the effect of protein extracted from a composition comprising Hermetia illucens that were subjected to extra purification steps performed by centrifugation and filtration;
**Figure 15** illustrates high molecular weight proteins by 1 dimensional (1 D) gel electrophoresis from stressed Hermetia illucens;

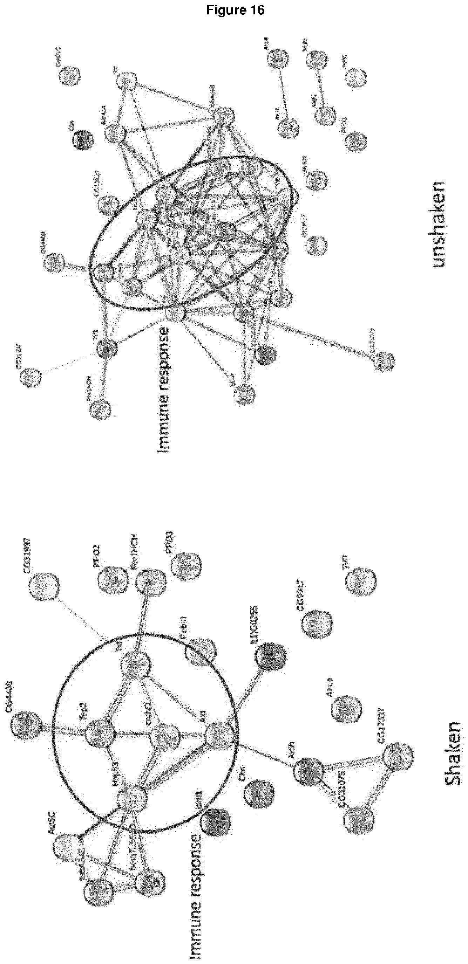

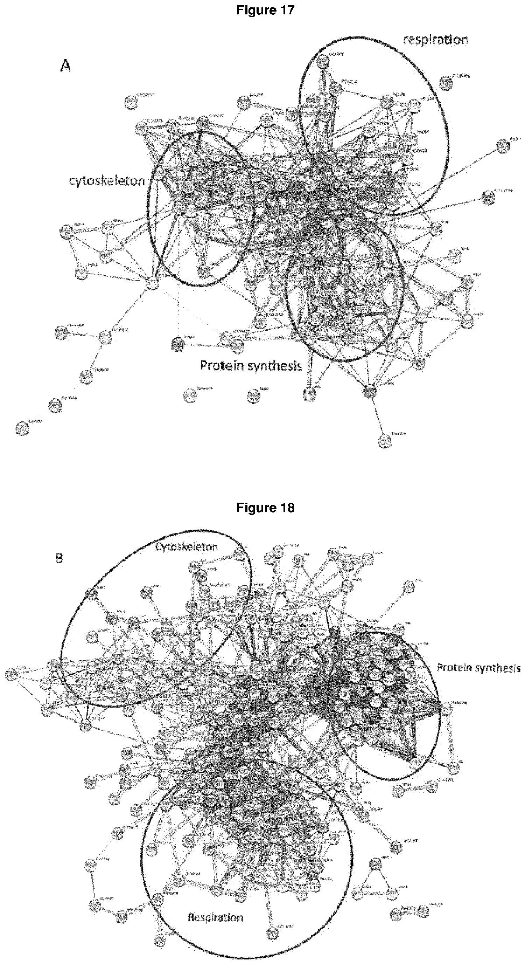

**Figures 16-18** illustrate protein interaction networks performed on a composition comprising Hermetia illucens had been stressed (S) and unstressed (U);
**Figure 19** illustrates antimicrobial activity of protein extracted from a composition comprising Hermetia illucens using organic solvent extraction methods;
**Figure 20** illustrates a HPLC chromatogram of proteins separated using a non-polar method;
**Figure 21** illustrates a HPLC chromatogram of proteins separated using a polar method;
**Figure 22** illustrates inhibition assays performed using fractions collected by HPLC;
**Figure 23** illustrates HPLC chromatograms of protein extracted from a composition comprising Hermetia illucens by methanol/water (90:10);
**Figure 24** illustrates a fraction (Fraction 12) had a distinctive yellow colour and had absorbance at 440 nm;
**Figure 25** illustrates a fraction (Fraction 12) had some activity against *E*. *coli;*
**Figures 26** **and** **27** illustrate HPLC chromatograms of protein extracted from a composition comprising Hermetia illucens;

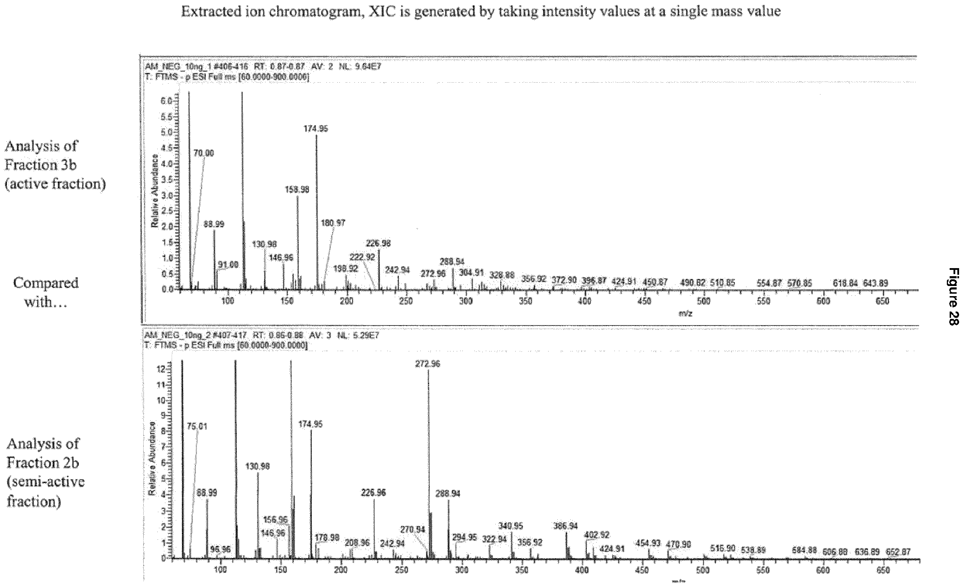

**Figure 28** illustrates an extracted ion chromatogram generated by taking intensity values of a ingle mass value;

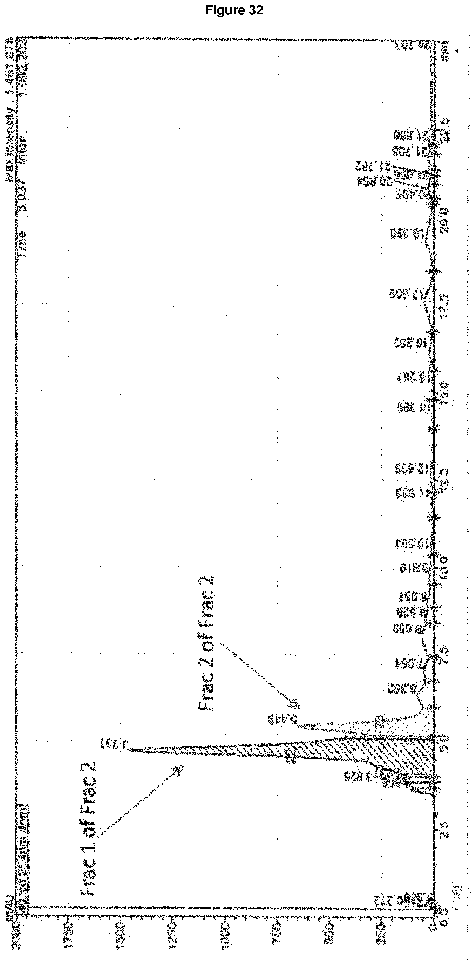

**Figures 29****,** **31****, and** **32** illustrate HPLC analysis of Fractions 1 and 2, which showed similar peaks with similar retention times;
**Figure 30** illustrates both Fractions 1 and 2 showed activity in ZOI assays;
**Figure 33** illustrates Fractions AM11, AM12, and AM14 had activity against all bacteria as shown in ZOI assays;

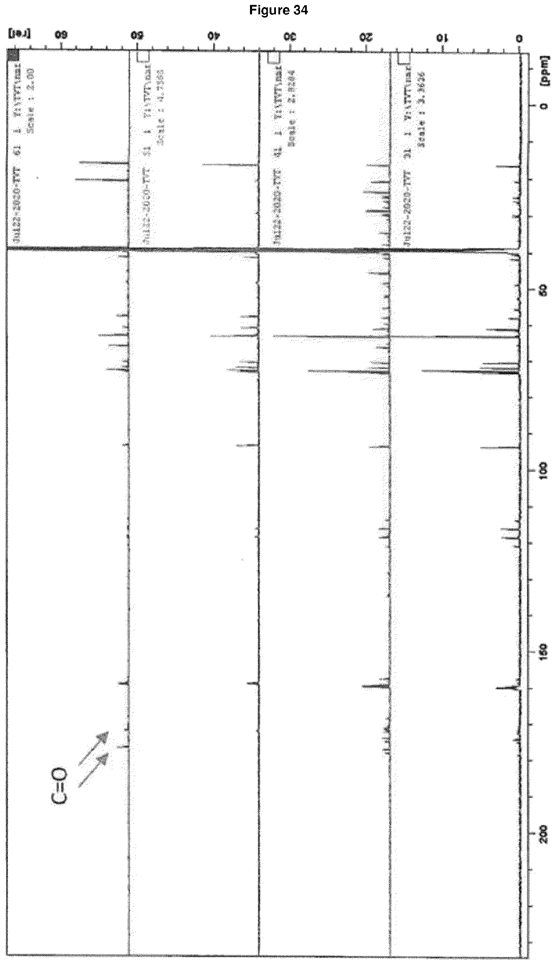

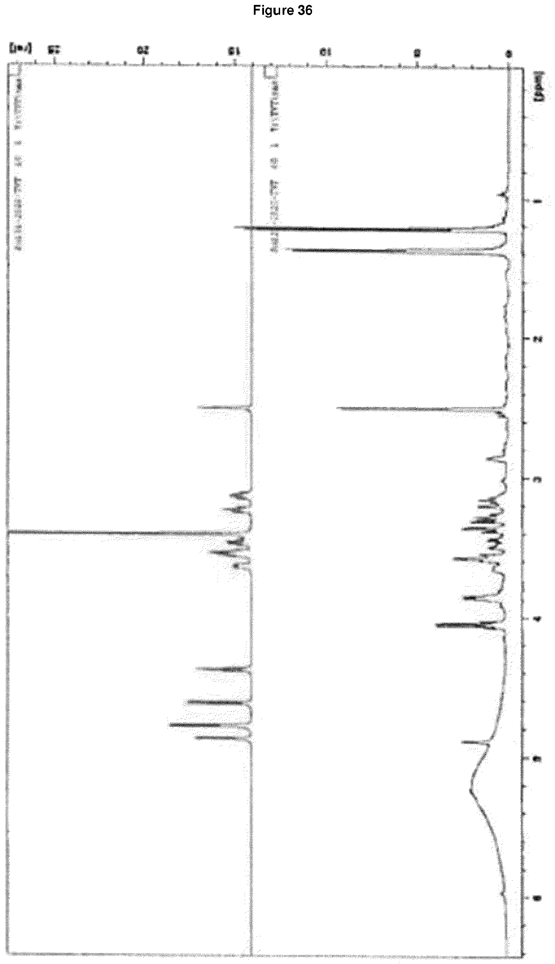

**Figures 34-37** illustrate NMR analysis performed on four samples; AM 11 to AM14. AM12 and AM14;

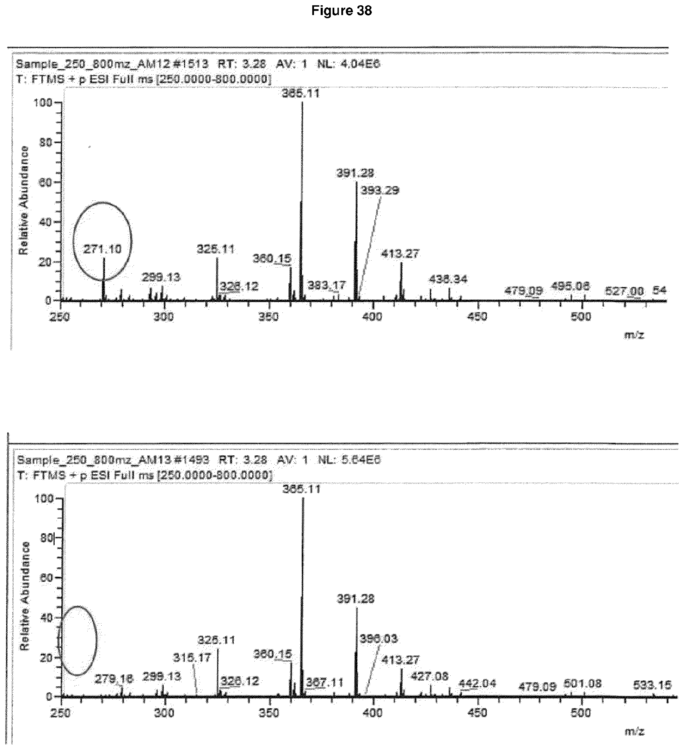

**Figure 38** illustrates peptides separated by an increasing acetonitrile gradient on a Hypercarb column using a 22-rninute reverse phase gradient; and
**Figure 39** illustrates the structural formula of the compounds identified.

### Examples

### Example 1

### Antimicrobial effect of a composition comprising Hermetia illucens

Four bacterial species were tested: *Escherichia coli* (E. coli), *Staphylococcus aureus, Methicillin resistant Staphylococcus aureus* (MRSA) and *Pseudomonas aeruginosa.* These species were inoculated from an agar plate using a sterilised inoculating loop and grown in an incubator for 36 hours in a nutrient medium at 37°C. 16 nutrient agar plates were made and four were inoculated with each bacterium species. The bacteria were diluted to a suitable concentration before being spread on the plates. *E.coli, Staphylcoccus aureus* and MRSA were serially diluted to a factor and pseudomonas was diluted to a dilution, the solutions were diluted with phosphate buffer saline (PBS). The bacterium was left to dry on the plate for a 2-5 minutes. Four different *Hermetia illucens* products were used while conducting this experiment, two wet samples and two dry samples. The wet samples consisted of an oil produced by pressing *Hermetia illucens* and an extraction of haemolymph from live *Hermetia illucens.* Due to difficulty in extraction of this haemolymph by conventional methods, 10 live larvae were crushed using a mortar and pestle with 5ml of PBS in order to obtain the product. The two dry samples consisted of a powdered meal produced by grinding dried *Hermetia illucens* and a frass that contains *Hermetia illucens.* Wells were made in the agar plates using the broad end of a pipette tip, these wells were filled with the above described products. A solution of ampicillin with a concentration of 25mg/ml was used as a control by which the rate of antimicrobial activity could be compared. In addition to the wells in the case of the dried products a sample was applied to the surface of the plate. The plates were then stored in an incubator at 37°C for approximately 17 hours before the results were taken.

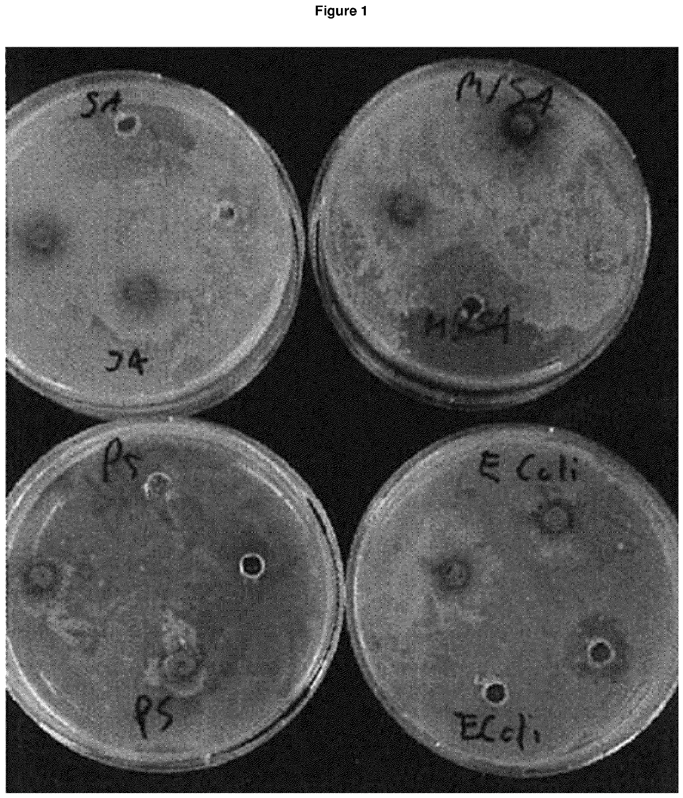

For the oil test, the only zone of inhibition was detected against the ampicillin control on the plate, indicating no or very little antimicrobial activity in the oil. In the quantity and concentration as seen in Figure 1, there are no zones around the test wells, indicating no antimicrobial effect.

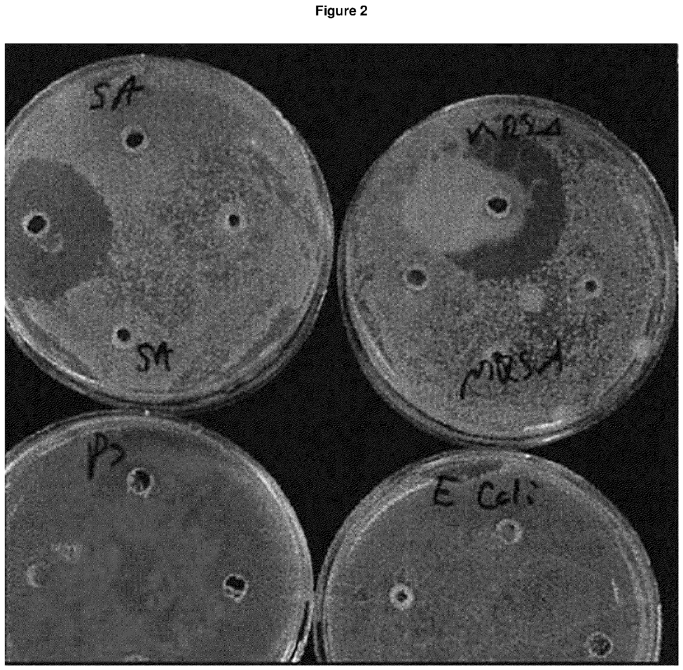

A similar result was seen in Figure 2 representing the haemolymph extraction test which showed no definitive zone of inhibition against any of the bacterial agents.

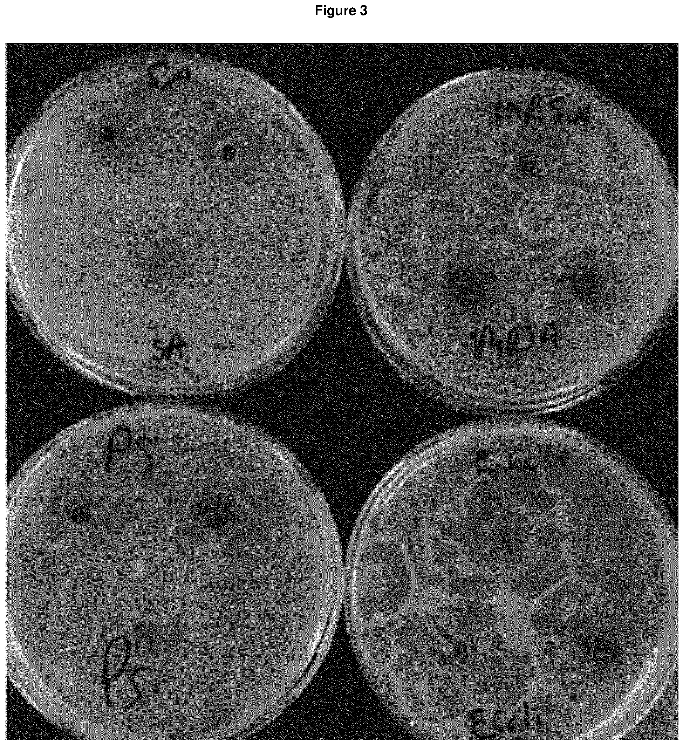

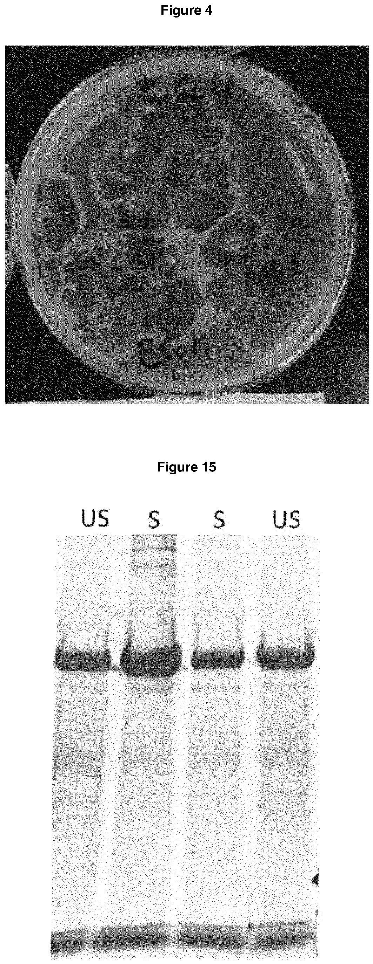

A positive result was seen in the frass testing and was shown to have a zone of inhibition on the E. *coli* but no other species tested. It is difficult to conclude if this was due to the antimicrobial activity or bacterial antagonism due to the presence of another bacterial species present in the product. This is shown in Figures 3 and 4. Figure 3 shows the full panel of frass testing where no other zones of inhibition were detected and Figure 4 is a closer look at the result formed on the E. *coli* test.

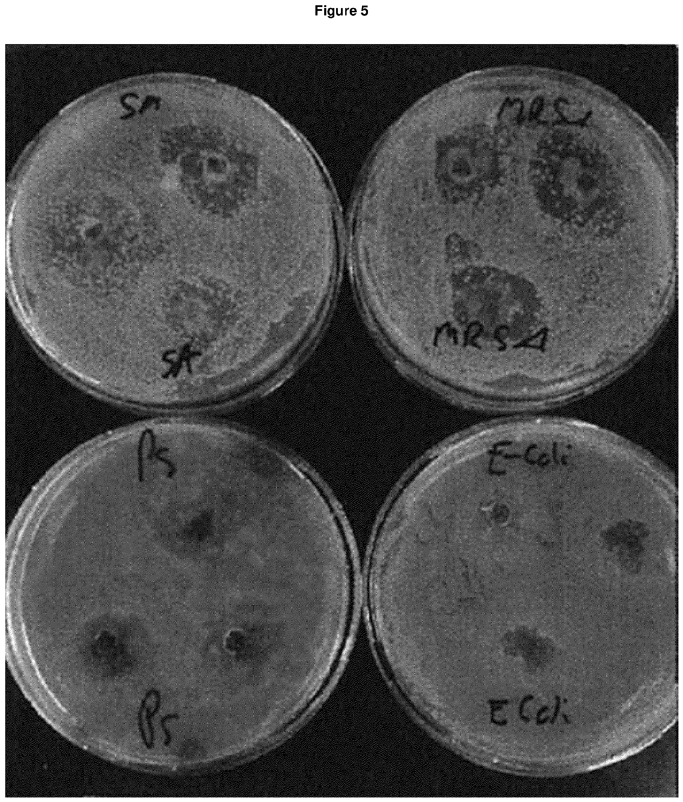

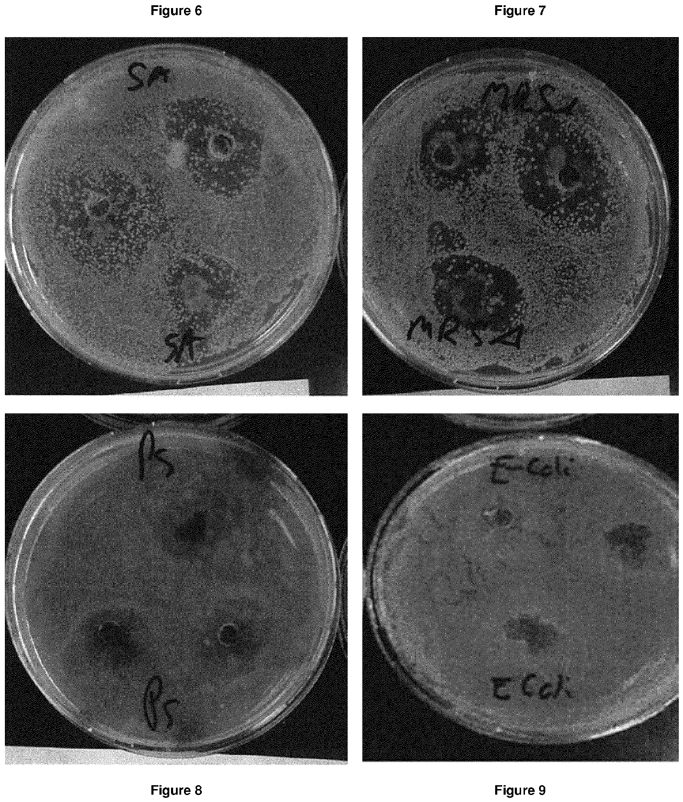

The most interesting result was that of the meal testing, which showed positive zones of inhibition against *Staphylococcus aureus, MRSA and Pseudomonas* but showed no effect against *E coli.* As seen in Figures 5-9, the zone of inhibition on the *Staphylococcus aureus* and *MRSA* plates are similar in size to that of the ampicillin control in the above testing. The zone of *Pseudomonas* inhibition in Figure 8 is considerably smaller but indicates some antimicrobial activity against this bacterium. The meal was the product with the highest level of antimicrobial activity.

The results from this example can be summarised in Table 1 below:

**Table 1: Summary of test results of Example 1**

| | ***Staphylcoccus aureus*** | ***E coli*** | ***Psuedomonas*** | ***MRSA*** |
|---|---|---|---|---|
| **Haemolymph** | - | - | - | - |
| **Oil** | - | - | - | - |
| **Frass** | - | + | - | - |
| **Meal** | + | - | + | + |

The results of this example are intriguing and promising and may serves as the initial step in the design and production of a viable alternative to antibiotic use. The comparison with ampicillin, the control, would indicate that the positive samples would be within the range of the potency of the antibiotic made at that concentration. The results of this example demonstrate that a composition comprising Hermetia illucens sufficiently out preformed any of the other samples tested giving clear zones of growth inhibition around the gram positive bacteria tested, *Staphylycoccus aureus, pseudomonas* and most impressively *MRSA.* The Frass sample interestingly showed a zone of inhibition around the gram negative species E-coli which was unaffected by any other products tested. This inhibition may have resulted from bacterial antagonism rather than antimicrobial activity as the zone was fragmented and not 100% void of microbes. It can be speculated that the antimicrobial activity of the samples may be coming from the antimicrobial peptides abundant in *Hermetia illucens* and other insect species due to their lifecycles in the soil among bacterial species. The testing of the meal was by far the most successful, resulting in zones of inhibition around both non-resistant and resistant strains of *Staphylococcus aureus* and showing inhibition around *Pseudomonas.* This species is difficult to treat by some convention antibiotics due to its thick mucosal membrane and despite its classification as gram negative bacteria. These results would suggest that the causative agent in the meal impacts or inhibits the growth of cell walls in bacterial cells by preventing cross-linking in the developmental stage this is similar to that of penicillin or beta lactam drugs.

The antimicrobial activity of a composition comprising Hermetia illucens against a range of bacteria and fungi was assessed using a well diffusion assay format. Bacteria included Staphylococcus aureus, methicillin resistant S. aureus (MRSA), Pseudomonas aeruginosa, Salmonella spp., Bacillus subtilis, Escherichia coli. Fungi used included the following: Candida albicans, Candida glabrata, Candida krusei, Aspergillus fumigatus and Penicillium spp. The proteomic response of selected Gram positive and Gram negative bacteria to a composition comprising Hermetia illucens was assessed in order to establish the likely mode of action of the meal.

### Example 2

### Proteomic characterization of a composition comprising Hermetia illucens

The antimicrobial peptides (AMPs) in a composition comprising Hermetia illucens were extracted and identified using label free quantitative proteomics. This technique enables the identification and quantification of AMPs in meal and gives the potential to compare the AMPs with AMPs from other species. Using this approach, it is possible to identify the possible mode of action of AMPs and ascertain the microbes against which they are active. Protein identification from the MS/MS data was performed using the Andromeda search engine in MaxQuant (version 1.2.2.5; http://maxquant.org/) to correlate the data against a 6-frame translation of the EST contigs for *Galleria mellonella.* Results processing, statistical analyses and graphics generation were conducted using Perseus v. 1.5.5.3. LFQ intensities were log2-transformed and ANOVA of significance and t-tests were performed using a p-value of 0.05 and significance will be determined using FDR correction (Benjamini-Hochberg). Proteins that had non-existent values (indicative of absence or very low abundance in a sample) were also be used in statistical analysis of the total differentially expressed group following imputation of the zero values using a number close to the lowest value of the range of proteins plus or minus the standard deviation. After data imputation these proteins were included in subsequent statistical analysis.

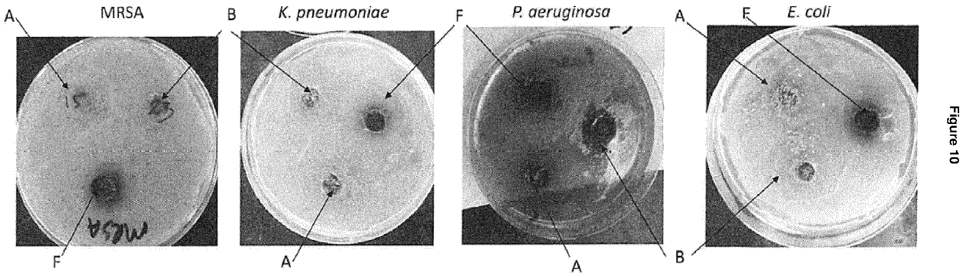

A composition comprising Hermetia illucens (ground, dehydrated Hermetia illucens meal) (A) and (B) and frass (F) derived from Hermetia illucens were crushed with liquid nitrogen and approximately 50 mg was placed into each well of an agar plate containing bacteria (∼100,000 bacterial cells per plate). The plates were incubated at 37°C for 24 hours. The results in Figure 10 show that the meal A produced the greatest zone of inhibition (i.e. a zone around which the bacteria will not grow due to antimicrobial activity) in MRSA, *Klebsiella pneumonia,Pseudomonas aeruginosa* and *Escherichia coli.* Oil was also tested but no zones of inhibition were observed.

Proteins were extracted from the meal samples and assessed for high molecular weight proteins by 1 dimensional (1D) gel electrophoresis, which separates proteins out based on size and charge. Proteins were detected by this method in the meal but not in the frass. The proteins from frass may to be small to be identified by this method.

Proteins extracted from meal (A), meal (B) and frass (F) were quantified and used to assess toxicity on a number of Gram-positive and Gram-negative bacteria. In meal A, 23.3 mg/ml protein was extracted and 100 uL of this was added to each well. In meal B, 29.9 mg/ml protein was extracted and 100 uL of this was added to each well. 1.87 mg/ml protein was extracted from frass and 100 uL of this was added to each well. The largest zones of inhibition were visualized around wells containing samples A on agar plates containing *MRSA, S. aureus, E. coli and C. albicans* (yeast). Protein from Meal A had the greatest antimicrobial effect on the bacteria and fungus tested in this assay.

In this example, two samples of a composition comprising Hermetia illucens and frass derived from Hermetia illucens were tested for antimicrobial activity against a small number of Gram-positive (MRSA and 5. aureus) and Gram-negative (E. coli, K. pneumoniae and P. aeruginosa) bacteria and the yeast (C. albicans). The dried material from the meal showed greatest anti-bacterial activity. No zones of inhibition were observed where the oil was used. Protein was extracted from both samples of meal and the frass. The proteins from the meal could be visualised by 1D gel electrophoresis but the frass could not, possibly because the proteins in the meal were larger and more abundant.

### Example 3

### Optimisation of methods for purification of a composition comprising Hermetia illucens

AMPs were extracted from a composition comprising Hermetia illucens using a variety of biochemical and analytical techniques. Initially, ammonium sulphate precipitation was used to concentrate peptides which were then be purified by HPLC fractionation. The bioactivity of fractionated samples was assessed using indicator microbes and fractions showing the most activity were further purified either by HPLC fractionation or by AKTA gradient fractionation.

Protein was extracted from meal (A) and (B) and subjected to extra purification steps performed by centrifugation and filtration. Large molecular weight proteins were visualized by 1D gel electrophoresis. The protein concentration reduced 15-fold (1.5 mg/ml protein was extracted from filtered meal (A) and (B) compared to 23-29 mg/ml extracted from unfiltered meal (A) and (B). However, it still retained the antimicrobial activity as can be seen by the zones of inhibition created when applied to agar plates containing MRSA *and E. coli.*

Proteins were purified by centrifugation and filtration. Although the protein yield was less than unfiltered proteins, the antimicrobial activity was retained and observed against *E*. *coli* and MRSA.

### Example 4

### Effect of stress on antimicrobial activity of a composition comprising Hermetia illucens

The immune system of insects is very sensitive to pathogens and various stresses and, in response, increases the production of AMPs in order to limit the development of pathogens. By subjecting insects to various physical and nutritional stresses, it is possible to significantly elevate the production of AMPs. In this example, Hermetic illucens larvae were subjected to physical stress (shaking, temperature change) and/or nutritional changes (i.e. different carbon sources) and the effect on the antimicrobial activity of the resulting composition comprising Hermetia illucens was assessed using a disk diffusion assay format. Optimum treatments to give elevated antimicrobial activity were identified.

To assess the changes in the protein profile of the haemolymph when larvae were stressed, larvae were shaken for two minutes and returned to the incubator (28°C). The haemolymph from shaken (S) and unshaken (US) larvae was extracted. The protein was quantified (100 mg/ml protein was detected in the haemolymph) and assessed for the presence of high molecular weight proteins by gel electrophoresis.

High-resolution mass spectrometry-based proteomics was used to characterize the protein profile of meal (A) and (B) and of the haemolymph from the shaken and unshaken samples. In meal (A), 124 proteins were detected. In meal (B), 335 proteins were detected. The difference in the number of proteins detected may be due to the differences in larval processing between meal (A) and meal (B). Nonetheless, similar proteins were detected in both samples.

**Table 2: Proteins extracted from larval haemolymph**

| Top 20 proteins from shaken larvae | Top 20 proteins from unshaken larvae |
|---|---|
| Phenoloxidase 2 | Phenoloxidase 2 |
| Actin-5C | Chitinase-like protein Idgfl |
| Phenoloxidase 3 | Actin-42A |
| Chitinase-like protein Idgfl | Tubulin alpha-1 chain |
| Thioester-containing protein 2, isoform G | Thioester-containing protein 2, isoform F |
| Angiotensin-converting enzyme | Tubulin beta-1 chain |
| Ferritin | Myosin heavy chain, isoform V |
| Transferrin | Angiotensin-converting enzyme |
| Uncharacterized protein, isoform A | Fructe-bisphphate aldolase |
| Fumarase 1, isoform B | Ferritin V |
| CathD, isoform A | Heat shock 70 kDa protein cognate 4 |
| RE42475p | RE42475p |
| FI17510pl | Glyceraldehyde-3-phphate dehydrogenase 1 |
| Cystathionine beta-synthase | Cystathionine beta-synthase |
| Heat shock protein 83 | FI17510p1 |
| Fructe-bisphphate aldolase | Uncharacterized protein, isoform A |
| Nucleome remodeling factor- 38kD, isoform C | Elongation factor 1-alpha 1 |
| Tubulin beta-1 chain | Major heat shock 70 kDa protein Ba |
| Myosin heavy chain, isoform S | FI05334p |
| Ejaculatory bulb-specific protein 3 | Enolase |
| Aldehyde dehydrogenase | Heat shock protein 83 |
| Tubulin alpha-1 chain | Chromatin-remodelling A TPase IN080 |

**Table 3: Proteins extracted from Meal A and Meal B**

| Top 20 proteins Meal (A) | Top 20 proteins Meal (B) |
|---|---|
| Myosin heavy chain | Myosin heavy chain |
| Tropomyosin | Tropomyosin 2 |
| ATP synthase subunit beta, mitochondrial | Actin |
| Actin | ATP synthase subunit beta, mitochondrial |
| Endoplasmic reticulum chaperone BiP | Tubulin Endoplasmic reticulum chaperone BiP |
| Arginine kinase | ATP synthase subunit alpha, mitochondrial |
| Troponin C, isoform 3 | Heat shock 70 kDa protein cognate 4 |
| Heat shock 70 kDa protein cognate 4 | Cofilin/actin-depolymerizing factor homolog |
| ATP synthase subunit alpha, mitochondrial | Arginine kinase |
| Cytochrome c-2 | Troponin C |
| Heat shock protein 68 | Calmodulin |
| Heat shock protein 60A | Tubulin beta-2 chain |
| Tubulin alpha-1 chain | Heat shock protein 60A |
| C-type lectin 27kD | Histone H4 |
| Cytochrome c oxidase subunit 5B, isoform A | Histone H2A |
| Ferritin | Histone H2B |
| Histone H4 | Histone H3 |
| 40S ribosomal protein S 14b | 40S ribosomal protein S7 |
| Voltage-dependent anion-selective channel | Cytochrome c oxidase subunit SB, isoform A |
| Gasp, isoform B | 40S ribosomal protein S14b |

Protein interaction networks were also performed on the haemolymph from the larvae that had been shaken (S) and unshaken (US). Between 30 and 35 proteins were detected in each sample. The results showed similar proteins in each sample, but as this was a qualitative rather than a quantitative test, it was not possible to determine whether the abundance of proteins were higher in one sample over the other.

Haemolymph was extracted from larvae that were stressed by shaking and from unshaken larvae. Protein was extracted from haemolymph and both meal samples and prepared for proteomic analysis by mass spectrometry. The results showed similar protein profile in the shaken and unshaken haemolymph and in both meal samples.

### Example 5

### Isolation of antimicrobial peptides from a composition comprising Hermetia illucens

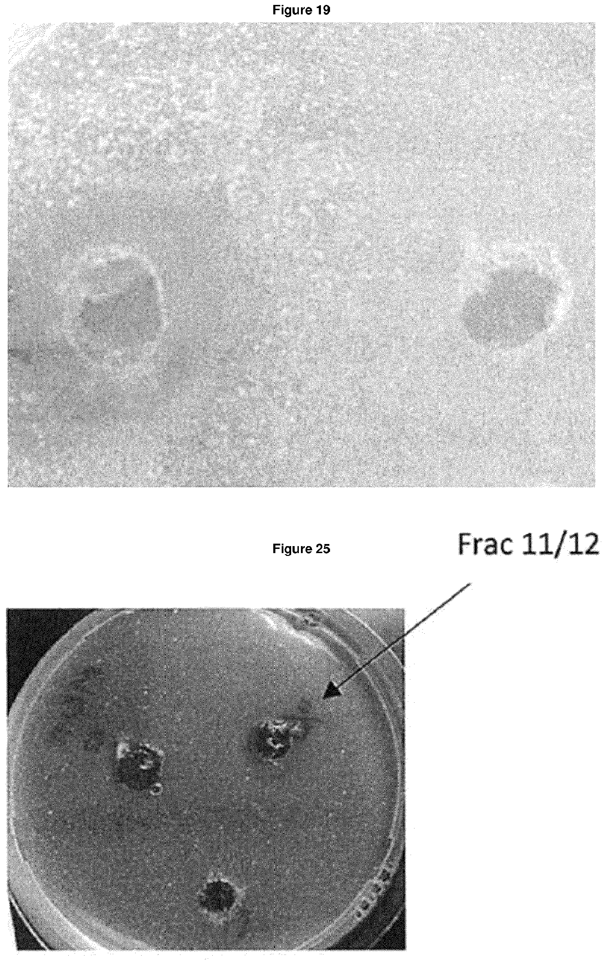

Samples of a composition comprising Hermetia illucens (ground, dehydrated Hermetia illucens meal) were mixed with methanol and water in a 9:1 ratio for 18 hours at 4°C with protease inhibitors. The aqueous solution was separated from the solid material by centrifugation at room temperature. The aqueous solution was dried down by rotary evaporation at 55°C under low pressure over 20-30 minutes. The dried material was suspended in deionized water (70%) and methanol (30%). Solid material was removed by centrifugation and the liquid was passed through 0.22µL centrifuge filters to remove any remaining contaminants. A toxicity assay was performed with this material (100µL) to verify antimicrobial activity (see Figure 19). Once activity was verified and the protein extraction method validated, the samples were prepared for HPLC.

The antimicrobial activity of protein extracted from a composition comprising Hermetia illucens using organic solvent extraction methods was assessed Protein (100µL) was added into the wells (A) of agar plates containing *E.coli.* To demonstrate that the antimicrobial activity was due to the protein and not the methanol, a control (70% water, 30% methanol) was added to the other wells (B). The lack of bacterial growth around the well containing protein extracted from a composition comprising Hermetia illucens demonstrates antimicrobial activity.

HPLC is a technique used to separate, identify, and quantify components contained within in a complex mixture. A sample is dissolved in a solvent (e.g. water or methanol) depending on the polarity (i.e. how soluble a substance is in water). Pumps pass pressurized liquid solvent (the mobile phase - e.g. water and acetonitrile) through a column containing solid adsorbent material (stationary phase- e.g. silica with carbon atoms attached, usually C18 or C8). As the sample is passed through the column, the components contained within the sample interact with the carbon atoms. The types of interactions are determined by the chemical characteristics of each component (e.g. polarity/water solubility). Because the components interact differently with the column, this leads to the separation of the components as they flow out of the column. Several factors influence the speed at which the components leave the column including; the chemical nature of the compound, the composition of the mobile phase and the type of stationary phase used. For example, where a C18 or a C8 column is used, polar molecules do not interact for long with the carbon atoms bound to the solid particles, and so they leave the column soon after they are applied. In contrast, non-polar compounds interact more strongly with the carbon atoms and so the time at which they leave the column is far later than polar compounds. The time at which each component emerges from the column is referred to as the retention time. The retention time is important, as it is an identifying characteristic of each component. The type of column used can affect the retention time of a sample component. To visualize the separated compound bands as they emerge from the column, information is sent from a detector to a computer, which generates the chromatogram. Ultraviolet (UV) detectors measure the absorbance of light of fixed wavelength in the UV or visible wavelength range (between 190 and 400 nm) against a reference beam. Each component of the sample absorbs light at different wavelengths. The magnitude of the absorbance is related to the concentration of the substance. UV absorbance is an important indicator of the chemical structure of a compound.

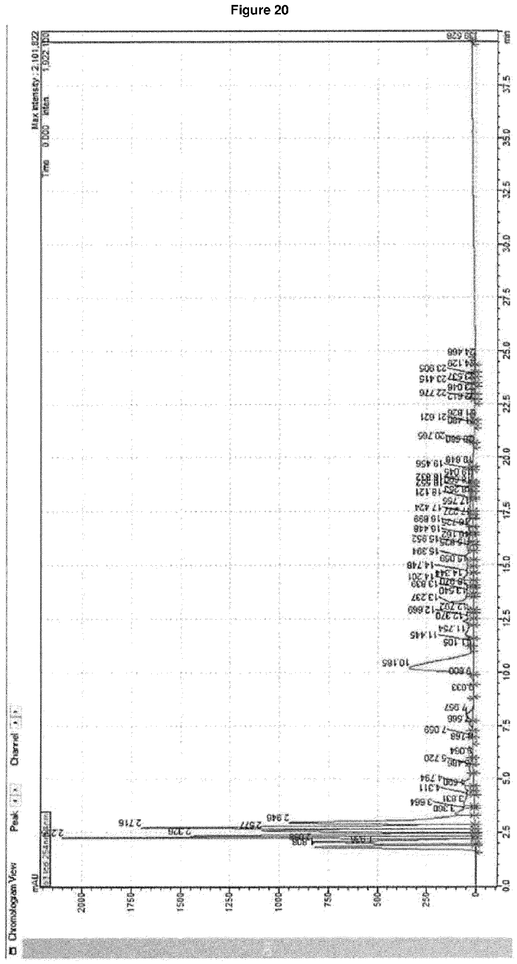

In this example, several HPLC protocols were initially tested to analyse the proteins extracted from a composition comprising Hermetia illucens. Initially, a protocol to investigate the polarity and the degree of protein separation was investigated. A mobile phase suited to non-polar compounds was used (see Figure 20). This involved pumping a mixture of water and an increasing gradient of acetonitrile (a non-polar solvent) through the column along with the sample. A lot of material was detected coming off the column quite early (i.e. the retention time was between 1 and 5 minutes) and the separation of the compound was quite poor (notice the peaks are clustered together at the beginning of the chromatogram) (see Figure 20). This suggested that the compound contains several components of a polar nature, as they did not interact with the C 18 column for long before being eluted off the column (see Figure 20). The chromatogram detected several components within the mixture that were less polar, as indicated by their retention time (i.e. 10 minutes).

Referring to Figure 20, there is shown a HPLC chromatogram of proteins separated using a non-polar method. Notice the main peaks are eluted from the column very quickly. One peak was detected at 10 minutes suggesting the polarity of this was less than the other components.

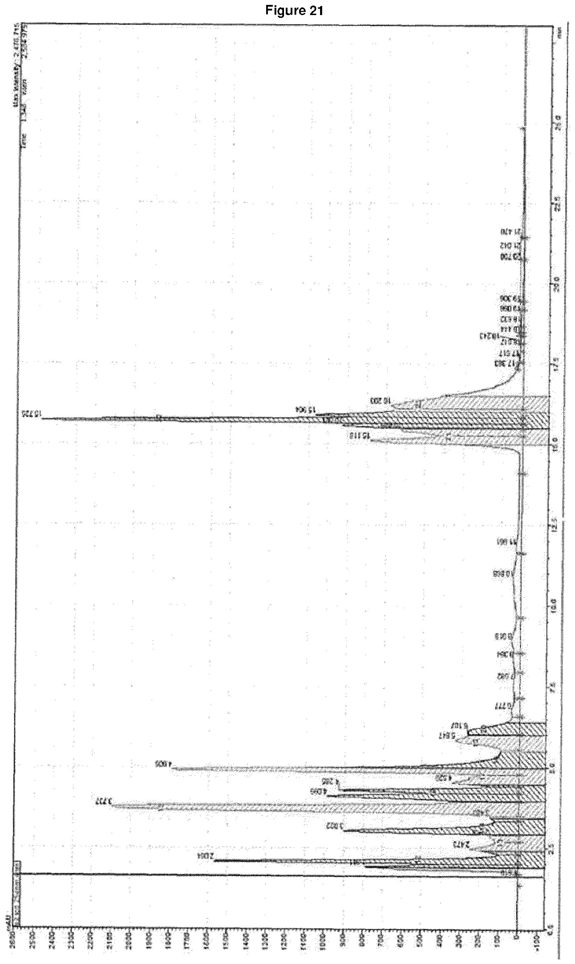

With this in mind, a protocol was employed to separate polar and non-polar components of the mixture. This involved using water only as the mobile phase for the first 15 minutes. During this time, the polar components interact with the column long enough to be detected before they are eluted off the column. Compared to the previous method used, this protocol also allowed for better separation of the protein components. A non-polar solvent, acetonitrile, was gradually introduced into the mobile phase. This interacts with the non-polar components of the sample and these are detected as they are eluted from the column. In this way, the separation of both polar and non-polar components could be achieved (see Figure 21). The peak visualized at 10 minutes in Figure 20 is likely to be that visualized at 15-16 minutes in Figure 21.

Referring to Figure 21, there is shown a HPLC chromatogram of the components from the protein mixture separated using the polar method. Peaks are well resolved and interact for longer periods with the column because of the method used to analyse this material.

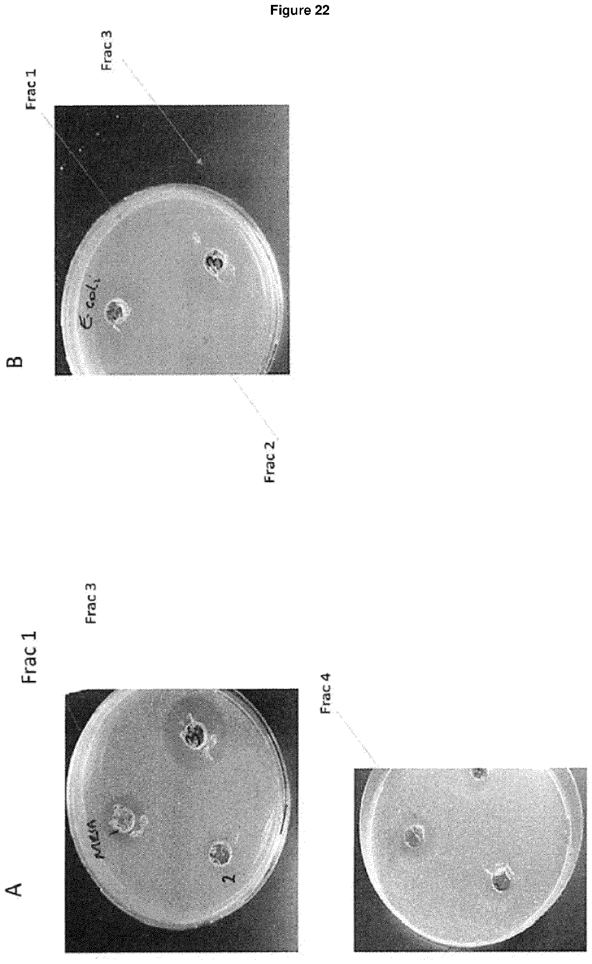

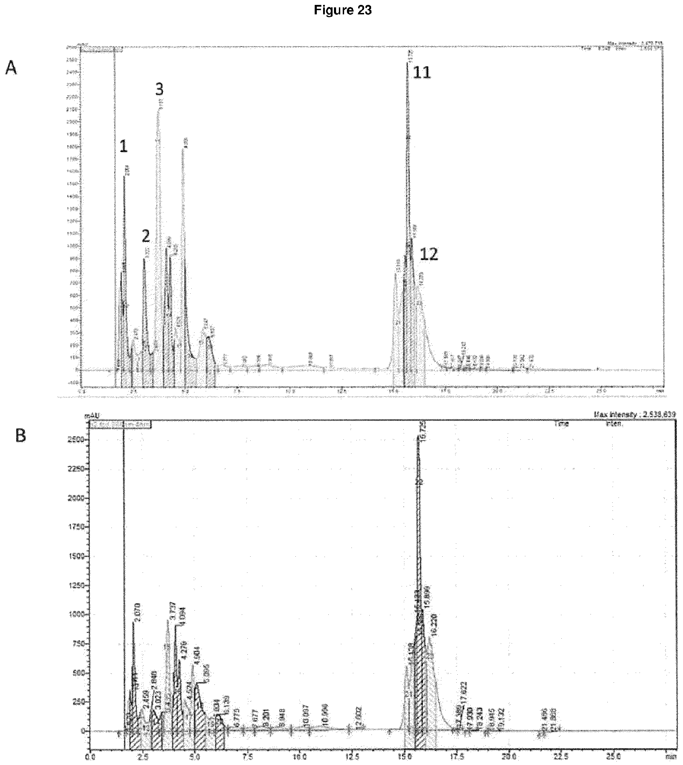

The decision of which fractions (parts of the sample) to collect was based on the peaks detected by HPLC (see Figure 21). The majority of the peaks detected were components that had eluted off the column between 1.5 and 6.5 minutes (polar components) and between 15 and 16.5 minutes (non-polar components). Fractions were collected (0.5 ml each fraction 1 - 12) until there was 40 ml in total. This material was lyophilized (i.e. the moisture was removed). The solid material was dissolved in 400µL water. Toxicity assays were performed on *E. coli* and MRSA using 100µL from each dissolved fraction. Fraction 3 showed the most activity, although fraction 1, 2 and 4 also showed some activity (see Figure 22). This suggests more than one component has antimicrobial activity or there was some carry-over in the fractions. These fractions had high absorbance at 254 and 280 nm (see Figure 23).

Referring to Figure 22, there is shown inhibition assays performed using fractions collected by HPLC. Fraction 1, 2 and 3 showed the greatest inhibitory effect on MRSA (A) and *E. coli* (B).

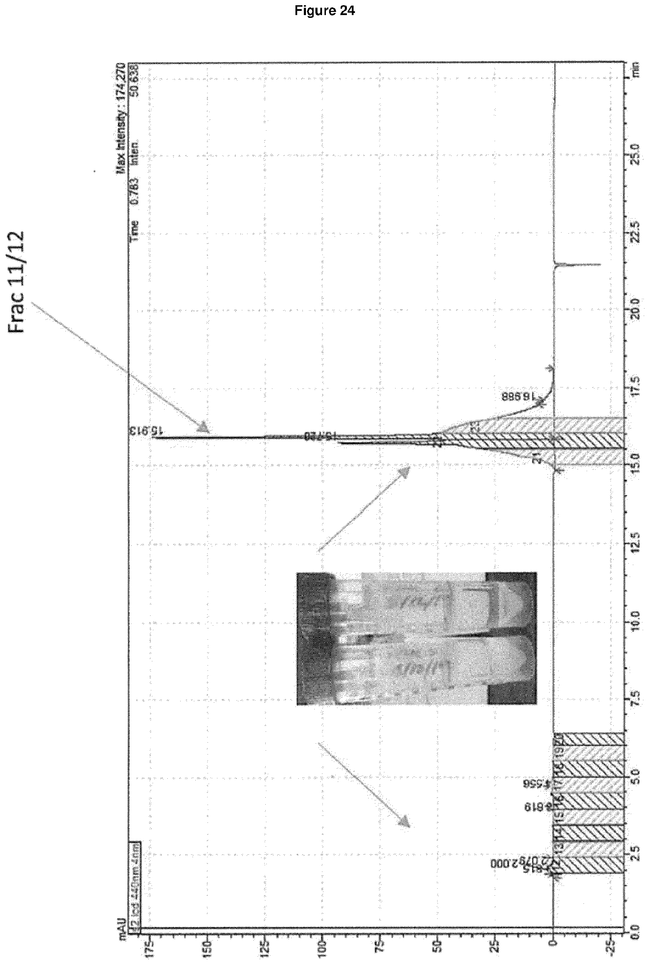

Figure 23 illustrates HPLC chromatograms of protein extracted from a composition comprising Hermetia illucens by methanol/water (90:10). Chromatogram A shows absorbance at 254 nm. Chromatogram B shows absorbance at 280 nm. Fractions were collected and lyophilized. Inhibition assays were performed with each fraction. Fraction 12 had a distinctive yellow colour (see Figure 24) and had absorbance at 440 nm. This fraction had some activity against *E*. *coli* (see Figure 25).

Referring to Figures 24 and 25, Fraction 11/12 (C) had some effect on *E. coli.* This fraction was yellow, and had absorbance at 440 nm suggesting a proline group. The other fractions did not absorb light at this wavelength and thus peaks are not detected in this chromatogram.

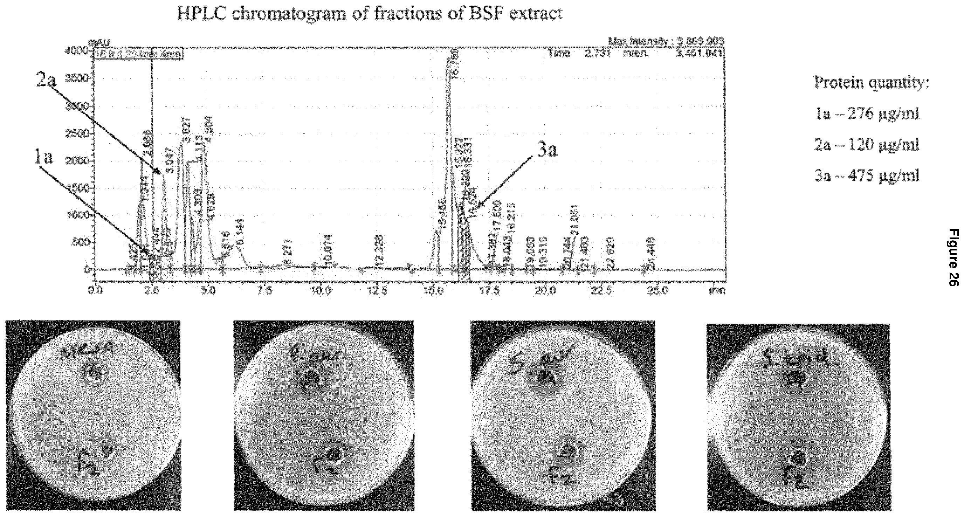

Referring to Figure 26, Fractions 1a and 2a of BSF extract (100µl) has activity against MRSA, P. aeruginosa, S. aureaus, and S. epidermidis. Fraction 3a had no activity and was not considered for further analysis.

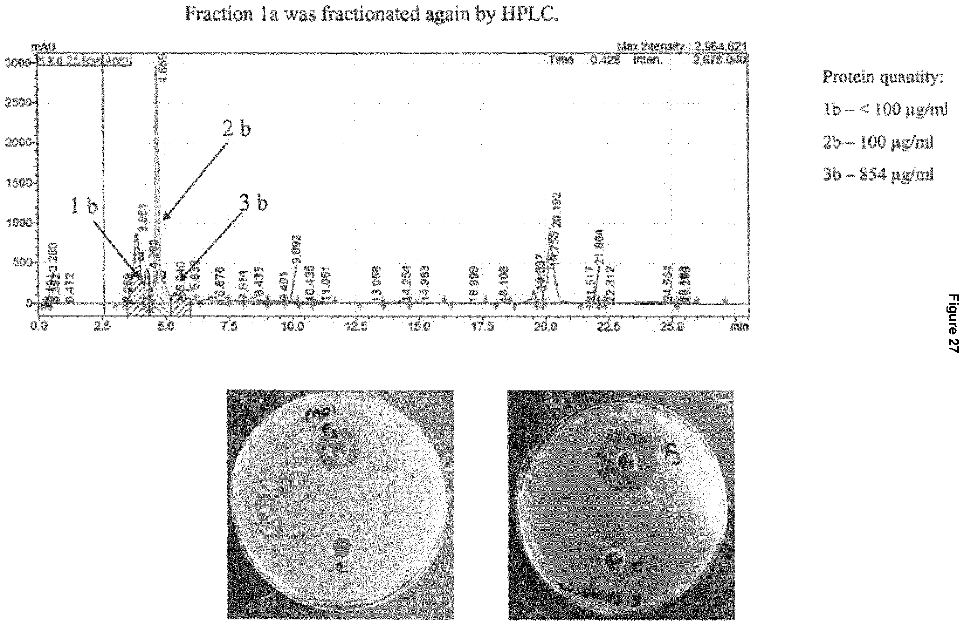

Referring to Figure 27, three fractions were collected. Fraction 3b (100µl) had the greatest amount of antibacterial activity when tested against P. aeruginosa and S. epidermidis.

Analysis by positive mode allows for the detection of positive ions (i.e. those with a plus charge). Analysis by negative mode allows for detection of negative ions (i.e. those with a negative charge). As we do not know the structure of the antibacterial compound, it is important to analyse the sample in positive and negative mode. Fraction 1 a- 3a and fractions 1 b and 2b were analysed by Mass Spectrometry in 1) positive mode and 2) negative mode.

### Example 6

### Analysis of the molecular structure of a composition comprising Hermetia illucens

Insect antimicrobial peptides (AMPs) are small (<100 amino acids), cationic molecules with activity against a wide range of bacteria. Many studies have analysed and characterized AMPs from the haemolymph of insect larvae but in the context of antibacterial activity, fewer studies have analysed cuticle-derived AMPs. This may be in part, due to the complex nature of the insect cuticle. The larvae of *Hermetia illucens* (Black soldier fly/BSF) contains many well-characterized antimicrobial peptides, which it has in common with other insects. In this example, a novel antimicrobial peptide detected in the cuticle of the larvae is disclosed. This peptide has activity against a range of Gram positive and Gram negative bacteria. Purification of the peptide was performed by RP-HPLC. Structural characterization of the peptide was performed by proteomics and NMR. Size filtration determined the peptide to be less than 10 kDa, which by comparison to known insect AMPs, is quite small. The peptide extraction, purification and characterization techniques are described herein.

A composition comprising Hermetia illucens (ground, dehydrated Hermetia illucens meal) was added to a glass reagent bottle containing hexane (25g/200ml) with phenylmethylsulfonyl fluoride (PMSF) (1mM/ml) and pepstatin A (200mg/ml). The mixture was separated into 50 ml falcon tubes and homogenized with a sonication probe. Homogenized material was returned to the glass bottle and left on a rocker at 4°C overnight. The contents of the glass bottle were let separate for one hour. The solvent (hexane) was removed with a Pasteur pipette to avoid disturbing the meal. A solution of methanol and deionized water (200ml, 90:10 ratio) containing PMSF (1mM) and Pepstatin A (200mg/ml) was added to the meal. The material was mixed and separated into 50ml falcon tubes and sonicated as before. The homogenised suspension was retumed to the rocker and left incubate over night at 4°C. The following day, the suspension was separated into falcon tubes and the solvent containing the peptide mixture was separated by centrifugation (3000 rpm for 20 minutes). The solvent was removed and the centrifugation step was repeated to ensure no particles were left in the solvent. Solvent (50ml) was dried by rotary evaporation under low vacuum at 65°C. The dried material was suspended in deionized water and methanol (80:20 ratio). This was repeated until all of the solvent that was separated from the solid material was dried. In general, from 200ml starting material, 20ml material was collected after rotary evaporation was completed. This material was passed through centrifugation filters with 0.22µm pores. The material was stored overnight at -20°C.

Protein quantity was determined by Qubit^{™} assay. In general, between 0.85 and 1.0mg/ml protein was detected from samples collected using this protocol. Antibacterial activity was determined by zone of inhibition assays. Bacteria (25/OD 1.0) were added to 25ml nutrient agar (hand hot). The agar was poured into plates. When the agar was set, wells were cut from the agar using a 1 ml pipette tip. 100µl of the extract was added to the wells. Controls were water/methanol, (80:20 ratio). The agar plates were incubated over night at 37°C.

Antimicrobial activity was determined by the presence of a clear zone around the wells containing the meal extract. Meal extract was purified by RP-HPLC (Shimadzu). The mobile phase (solvent A) was deionized water with 0.1% (trifluoroacetic acid) TFA and (Solvent B) was acetonitrile with 0.1 % TFA. The gradient conditions were:
0-10 minutes 10% acetonitrile
10-18 minutes 100% acetonitrile
18-20 minutes 100% acetonitrile
20-28 minutes 0 % acetonitrile.

Meal (40ml) was injected onto a Lunar Omega, 5µm polar C18, LC column (Phenomenex) at a flow rate of 0.5 ml/min. The fraction collector was set to collect fractions (0.5 ml) between 4.5 minutes and 6.5 minutes.

Fractions 1 and 2 were pooled together in glass vials and stored at -20°C. When all of the meal extract (20 mL) had been separated, the fractions were separated based on size. Fractions were passed to centrifuge filters with a molecular weight cut off of 10,000 Da (10kDa). Zone of inhibition assays showed that the fractions that had activity were less than 10kDa.

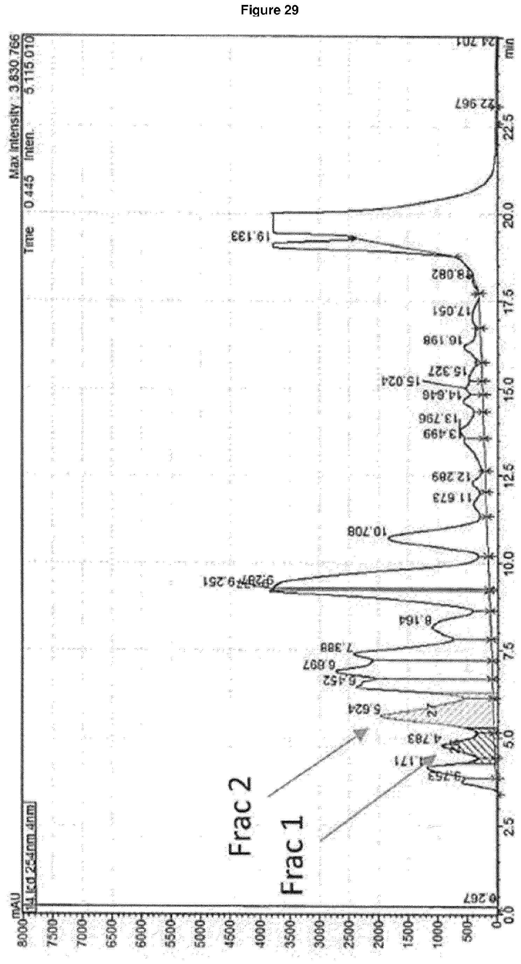

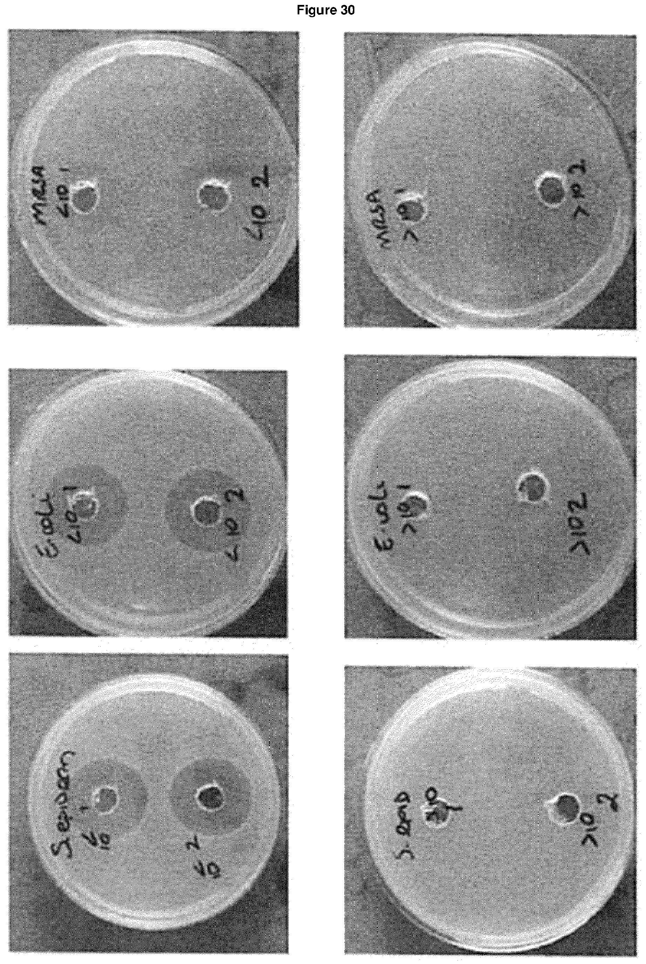

Fractions were freeze dried over four-five days. The volume of pooled fractions was 150ml. The lyophilized material was suspended in deionized water (5rnl). The concentrated material was quantified by Qubit. In general, Fraction 1 (collected between 4.5 and 5.5 minutes) contained 180-*200*µ*g*/*ml* protein. Fraction 2 (collected between 5.5 and 6.6 minutes) contained 300-330µg/ml protein. Both fractions showed activity in ZOI assays (see Figure 30). When each fraction was analysed separately, HPLC analysis of Fractions 1 and 2 showed similar peaks with similar retention times (see Figure 29). As such, parameters were set on the HPLC to optimize the amount of product collected altogether while maintaining the level of purity of the fractions collected. The conditions set for the collection of fractions for Fraction 1 were as follows:
Flow rate: 0.5 ml/min
0-10 minutes 0% acetonitrile
10-18 minutes 100% acetonitrile
18-20 minutes 100% acetonitrile
20-28 minutes 0% acetonitrile.

Fractions (0.35mL) were collected between 4.6- 5.3 minutes and between 5.3-6.0 minutes.

The conditions set for the collection of fractions for Fraction 2 were as follows :
Flow rate: 0.5 rnl/min
0-10 minutes 5% acetonitrile
10-18 minutes 100% acetonitrile
18-20 minutes 100% acetonitrile
20-28 minutes 0% acetonitrile.

Fractions (0.5ml) were collected between 4.5-5.5 minutes and between 5.5- 6.5 minutes. Fractions were pooled together as described previously and lyophilized.

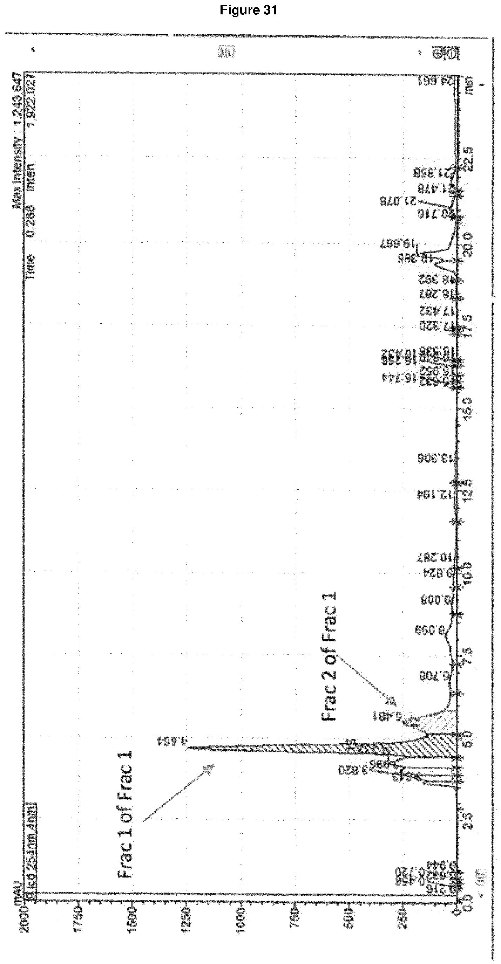

The material was fractionated by HPLC using a 10% acetonitrile gradient at the beginning of the run to ensure removal of glucose. Material was lyophilized and each fraction was tested again on bacteria. The active fraction was fractionated further, using a water only gradient. The three fractions (AM10, AM11 and AM12) were collected and tested for antimicrobial activity. A11 and AM12 were determined to have activity with AM12 showing the most activity as evidenced by the larger zone of inhibition (see Figure 29), NMR (see Figure 30) and MS (see Figure 31) was performed on five fractions collected from different batches; Batch 1, AMI10 to AM12 and Batch 2, AM13 (non-active) and AM14 (active).

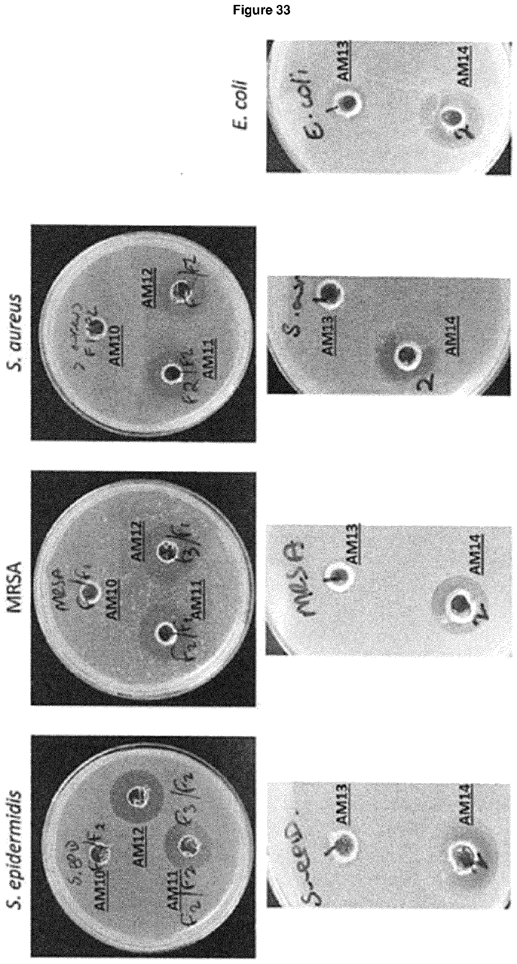

Referring to Figure 33, compounds were tested for activity against a range of gram positive and gram negative bacteria including S. epidermidis, MRSA, S. aureus, and E.coli. Compounds labelled AM11, AM12, and AM14 had activity against all bacteria as shown by the lack of growth surrounding the wells in the nutrient agar plates.

NMR analysis was performed on four samples; AM 11 to AM14. AM12 and AM14 had most antimicrobial activity. AM13 had no antimicrobial activity. AM11 had some activity, but less than AM12 and AM14. NMR analysis was performed with deteriorated water and DMSO. Comparative analysis of the chromatograms identified the presence of several peaks that were exclusive to AM12 and A14 (active fractions). DEPT 13C NMR identified at least two carbonyl atoms in AM12 and AM14 (178, 172 ppm), indicating the presence of amino acids.

Peaks detected in 1H NMR analysis in the aliphatic region indicate the presence of two alanine residues; one being a terminal alanine and one alanine adjacent to another residue, e.g. serine. Distinct peaks were not detected in the aromatic region of the bioactive compounds indicating that the derivative compound of interest does not contain amino acids containing benzene rings (e.g. tyrosine, histidine) and does not have properties relating to quinones.

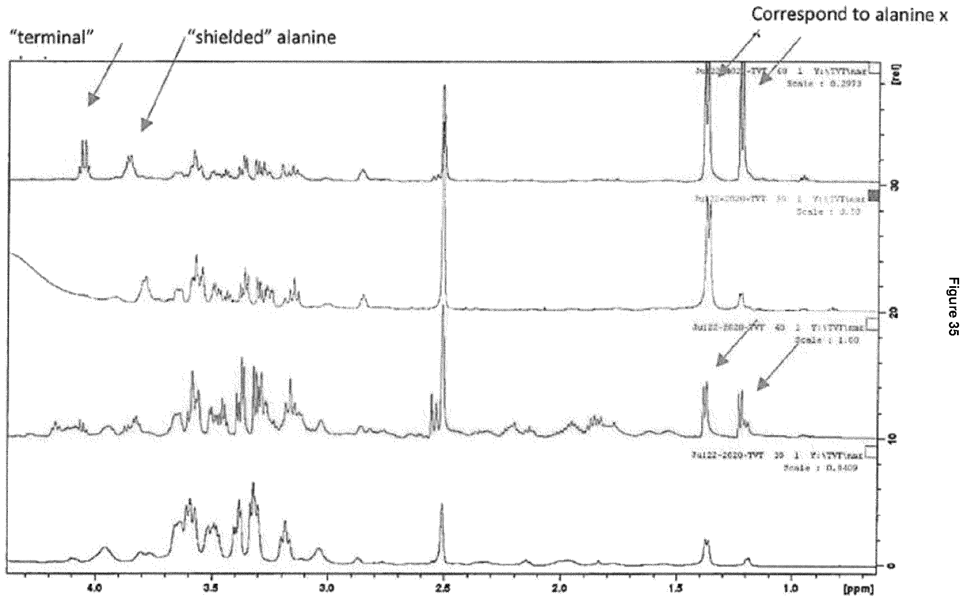

Figure 35 illustrates 1H NMR performed in DMSO; AM11: blue; AM12: red; AM13: green; AM14: purple. Two peaks identifiable in AM12 and AM14 are indicative of alanine residues. The same patterns are not present in AM 13.

An anomeric carbon indicates the presence of a sugar molecule. Trehalose, an abundant carbohydrate found in insect cuticles, has been ruled out by NMR. It is possible that the anomeric carbon atom belongs to a mannose residue:
Figure 37 illustrates DEPT 13C NMR performed in DMSO; AM11: blue; AM12: red; AM13: green; AM14: purple, wherein:
Peak at 93 ppm indicates an anomeric carbon for a glycoside;
Peaks at 73, 72, 71, 69 ppm indicate the C-H bond in the glycoside;
Peaks at 60 and 49 ppm indicate the alpha carbons from amino acids;
Peaks at 62, 60 and 57 indicate three CHz; one from a glycosyl and the other from another amino acid residues, possible a serine, glycine or asparagine; and
Peaks at 19, 15 indicate CH3-methyl carbons from alanine.

Sample material was suspended in water/formic acid (0.1%). Samples (200ng) were loaded onto a QExactive (ThermoFisher Scientific) high-resolution accurate mass spectrometer connected to a Dionex Ultimate 3000 (RSLCnano) chromatography system. Peptides were separated by an increasing acetonitrile gradient on a Hypercarb column (Thennofisher), using a 22-rninute reverse phase gradient at a flow rate of 250 nL/rnin-1. Some differences were observed between the various samples.

Fragmentation of the m/z 271.1 peak yielded a number of ions that have an m/z corresponding to the amino acids detected in the NMR data including alanine (m/z 88.04). It is possible that the m/z 271.1 peak represents Seryl-alanyl-alanine, i.e. serine, alanine, alanine, although not in this order. This may be the unglycosylated fragment of the over-all bioactive agent.

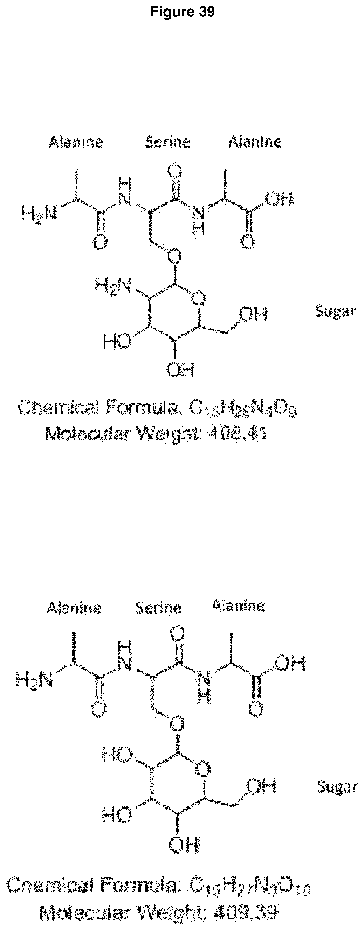

In summary, the bioactive agent appears to be a glycosylated tripeptide. While the precise type of sugar moiety is unknown, the NMR data suggests that this is a mannose. The peptides here are likely to be alanine and serine residues and may form a tripeptide. Including the sugar moiety, the glycosylated tripeptide may appear structurally similar to the compounds illustrated in Figure 39.

Antimicrobial peptides (AMPs) are integral to the insect immune response by providing defence against bacterial and fungal pathogens. The insect immune system and associated AMPs have been well characterized and many AMPs are widely conserved across the different species. By definition, AMPs are peptides, consisting of less than 100 amino acid residues. The peptides are cationic in nature, allowing the peptides to interact with the negatively charged outer membrane of the microbial cell. This interaction disrupts the cell membrane thereby killing the cell. The production of AMPs (e.g. cecropins and defensins) is induced upon infection and AMPs are released into the haemolymph of the insect where they act on the pathogen. The insect cuticle is the first line of defence against external pathogens. While many studies have focused on the isolation of AMPs from haemolymph (insect blood), few have analysed the cuticle as a source of AMPs. As we enter the era of antimicrobial resistance, much attention is being given to insects as a source of AMPs. AMPs extracted from insects have shown activity against a range of fungal and bacterial human pathogens. Thus, insects may contribute to the next generation of antimicrobial agents by providing an alternative to the range of current antimicrobial therapeutics, many of which are expected to become redundant in the coming years due to the rise in antimicrobial resistance.

As an ecological decomposer of organic matter, Hermetia illucens (Black Soldier Fly/BSF) is exposed to a range of potentially pathogenic fungi and bacteria. Thus, survival of the insect in this environment is dependent upon an extremely efficient immune system. The present invention has identified antibacterial activity in the cuticle of the larvae produced by BSF. The invention further relates to a protocol to extract proteins and/or peptides from the cuticle of the larvae, test the activity of these compounds on a range of fungal and bacterial pathogens, and isolate, purify and characterize the bioactive agent. The BSF cuticle was provided in the form of ground, dehydrated Hermetia illucens meal and therefore no further processing of the larvae was required. Initially, a series of extraction protocols were performed on the meal in order to isolate bioactive material from the BSF larvae. The protocol most effective at achieving this was optimized to achieve high yields and a pure compound.

In an embodiment, the ground, dehydrated Hermetia illucens meal was weighed and mixed with hexane in a 1:8 ratio (g:ml). Protease inhibitors were added to the suspension. The mixture was homogenized by sonication and incubated on a rocker overnight to remove lipids. The suspension was rested for one hour until the solid material and the hexane had separated. The hexane was removed. Methanol and water (200ml) (90:10) ratio was added with protease inhibitors. The meal was homogenized again by sonication and incubated overnight on a rocker. Following this, the suspension was transferred into tubes and the solid material was separated by centrifugation. The solid material was discarded. The methanol/water solution was dried under a vacuum by rotary evaporation. The dried material was suspended in water/methanol (90:10). This material was centrifuged to separate large particles and passed through 0.2µm pore filters before being stored at - 20°C.

Prior to separation by RP-HPLC, the material was centrifuged. The supematant was passed through 0.2µm centrifugal filters. The supematant was loaded onto a HPLC column (50µl at a time). Several fractions were collected. This fractionation procedure was repeated until 50ml of each fraction was collected. The fractions were freeze dried to produce a solid material. This was suspended in water. Each fraction was tested for activity against a range of fungal and bacterial pathogens using the zone of inhibition assay (ZOI assay). The size of the clear zone around the well in which the fractions were contained, indicated the level of activity against the pathogen.

The results showed that fractions eluting from the HPLC column between 4.5 and 6 minutes showed the greatest activity against a range of bacteria (MRSA, S. aureus, S. epidermidis, P. aeruginosa, E. coli, K. pneumonia, S. typhimurium, S. maltophilia and E. meningoseptica). The fractions did not show activity against the fungal pathogens Candida albicans and Aspergillus fumigatus. Futher fractionations were performed on the bioactive fractions to achieve a purer compound. These fractions were passed through centrifugal filters with a molecular weight (mw) cut-off of 10 kDa. The components of the fractions with a mw of <10 kDa retained antibacterial activity while those with a mw of >10 kDa did not. This indicates that the bioactive agent is a low molecular weight compound.

Comparative studies were performed by NMR on bioactive and non-active fractions collected by HPLC. A series of one and two-dimensional NMR experiments, including 1H NMR, DEPT 13C, TOCSY and COSY were performed on bioactive and non-active fractions. These studies revealed the presence of two alanine residues and a sugar molecule, possibly a mannose residue, connected to another residue by an O-linkage. Because the pattern did not correspond to a threonine residue, it is likely that the sugar is bound to a serine residue, as O-linkages only occur on serine or threonine residues. The sugar is less likely to be connected to an N-linked residue, thereby ruling out the possibility that the other amino acid is asparagine. The peak corresponding to one of the alanine residues suggests that the alanine is being shielded by another residue, suggesting that the glycosylated serine is positioned between the two alanine residues. High resolution mass spectrometry analysis of the purified fractions identified peaks that were present in the bioactive fractions but absent in the non-active fractions. Further analysis of these peaks revealed an ion with a molecular weight corresponding to that of a tripeptide (m/z ∼ 250). The addition of a sugar molecule increases the molecular weight of the molecule to approximately 409.

Taken together, the results indicate that the antibacterial agent isolated from the ground, dehydrated Hermetia illucens meal using the protocol outlined herein is a glycosylated tripeptide composed of two alanine residues and a glycosylated serine residue. The significance of the alanine residues lies in its ability to interact with the chemistry of the bacterial outer membrane. The serine residue provides a site for glycosylation, and confers the hydrophilic properties observed in the compound. In general, glycosylation provides the compound with stability and is crucial for the antimicrobial activity of AMPs, in part due to the binding interactions that occur between the sugar residue and receptors on bacterial cell membranes. The labour and monetary costs associated with synthesizing glycosylated peptides are high, thus the ability to extract and purify such compounds from an inexpensive biological source provides an attractive alternative for the large scale production of such therapeutics.

To conclude, it is proposed that that antibacterial activity observed in the extract isolated from the ground, dehydrated Hermetia illucens meal is caused, in part, by the presence of a glycosylated tripeptide. This compound is active against a range of Gram positive and Gram negative bacterial pathogens. The structure of the compound likely contains an alanine bound to the C terminal and N terminal of a glycosylated serine.

## Claims

1. A composition comprising Hermetia Illucens or derivative compounds therefrom for use as a disinfectant, antiseptic, or antibiotic.

2. A composition comprising Hermetia Illucens or derivative compounds therefrom for use as a bactericide or fungicide.

3. A composition comprising Hermetia Illucens or derivative compounds therefrom for use in the treatment of a bacterial or fungal infection.

4. A composition for use according to Claim 2 or 3, wherein the bacterium is selected from the genus Staphylococcus, Pseudomonas, Salmonella, Bacillus, Escherichia, and/or Klebsiella or wherein the fungus is selected from the genus Candida, Aspergillus, and/or Penicillium.

5. A composition for use according to any one of Claims 1-4, wherein the composition comprises mature Hermetia Illucens, Hermetia Illucens larvae and/or Hermetia Illucens larval cuticle.

6. A composition for use according to any one of Claims 1-5, wherein the composition comprises Hermetia Illucens haemolymph.

7. A composition for use according to any one of Claims 1-6, wherein the composition comprises Hermetia Illucens frass.

8. A composition for use according to any one of Claims 1-7, wherein the composition comprises at least one peptide derived from Hermetia Illucens, wherein the at least one peptide is less than 100 amino acids in length.

9. A composition for use according to Claim 8, wherein the peptide comprises at least three amino acids and at least one monosaccharide molecule.

10. A composition for use according to Claim 8 or 9, wherein the peptide comprises two alanine residues and one serine residue and at least one monosaccharide molecule, wherein each alanine residue is covalently attached to the one serine residue, and wherein the at least one monosaccharide molecule is attached to the serine residue.

11. A composition for use according to Claim 9 or 10, wherein the monosaccharide molecule is a hexose molecule selected from allose, altrose, glucose, mannose, gulose, idose, galactose, and talose.

12. A composition for use according to Claim 9 or 10, wherein the monosaccharide molecule is an amino sugar molecule selected from mannosamine (2-amino-2-deoxymannose) and glucosamine (2-Amino-2-deoxy-glucose).

13. A method of preparing a disinfectant, antiseptic, or antibiotic composition, the method comprising the steps of:
(a) providing Hermetia Illucens;
(b) dehydrating the Hermetia Illucens; and
(c) processing the dehydrated Hermetia Illucens.

14. A method according to Claim 13, further comprising the step of physically stressing the Hermetia Illucens.

15. A disinfectant, antiseptic, or antibiotic composition comprising Hermetia Illucens or derivative compounds therefrom; or obtained by the method of Claim 13 or 14.
